# EUROPEAN PATENT SPECIFICATION

(11) **EP 1 309 625 B1**
(45) Date of publication and mention of the grant of the patent: **15.06.2005**
(21) Application number: 01962220.8
(22) Date of filing: 17.08.2001
(51) Int. Cl.: C07K 14/705, C12N 15/12, C07K 16/28, C12N 5/06, C12N 5/08, A61K 39/395, A61K 39/00, A61P 35/00

(54) **THERAPEUTIC COMPOUNDS FOR OVARIAN CANCER**
THERAPEUTISCHE VERBINDUNGEN GEGEN EIERSTOCKKREBS
COMPOSES THERAPEUTIQUES CONTRE LE CANCER DES OVAIRES

(30) Priority: 17.08.2000 US 226243 P
(43) Date of publication of application: 14.05.2003
(73) Proprietor: GENZYME CORPORATION, Cambridge, MA 01701-9322 (US)
(72) Inventor: NICOLETTE, Charles, A., Framingham, MA 01701 (US)
(74) Representative: Helbig, Christian, Dr. Dipl.-Biol.
(86) International application number: PCT/US2001/025708
(87) International publication number: WO 2002/014352

(56) References cited:
- WO-A-00/43511
- JOBERTY G ET AL.: "The cell-polarity protein Par6 links Par3 and atypical protein kinase C Cdc42" NATURE CELL BIOLOGY, vol. 2, no. 8, 17 July 2000 (2000-07-17), pages 531-539, XP002213278 & DATABASE NCBI [Online] Homo sapiens partitioning-defective 3 splice var, Database accession no. AF252293
- HSU C-T ET AL.: "Vaccination against Gonadotropin-releasing Hormone (GnRH) Using Toxin Receptor-binding Domain-Conjugated GnRH Repeats" CANCER RESEARCH, vol. 60, 15 July 2000 (2000-07-15), pages 3701-3705, XP002213279

## Description

### TECHNICAL FIELD

The invention relates to the field of therapeutic compounds useful against human ovarian cancer.

### BACKGROUND OF THE INVENTION

The recognition of antigenic epitopes presented by molecules of the Major Histocompatibility Complex (MHC) plays a central role in the establishment, maintenance and execution of mammalian immune responses. T cell surveillance and recognition of peptide antigens presented by cell surface MHC molecules expressed by somatic cells and antigen presenting leukocytes functions to control invasion by infectious organisms such as viruses, bacteria, and parasites. In addition it has now been demonstrated that antigen-specific cytotoxic T lymphocytes (CTLs) can recognize certain cancer cell antigens and attack cells expressing these antigens. This T cell activity provides a basis for developing novel strategies for anti-cancer vaccines. Furthermore, inappropriate T cell activation plays a central role in certain debilitating autoimmune diseases such as rheumatoid arthritis, multiple sclerosis, and asthma. Thus presentation and recognition of antigenic epitopes presented by MHC molecules play a central role in mediating immune responses in multiple pathological conditions.

Tumor specific T cells, derived from cancer patients, will bind and lyse tumor cells. This specificity is based on their ability to recognize short amino acid sequences (epitopes) presented on the surface of the tumor cells by MHC class I and, in some cell types, class II molecules. These epitopes are derived from the proteolytic degradation of intracellular proteins called tumor antigens encoded by genes that are either uniquely or aberrantly expressed in tumor or cancer cells.

The availability of specific anti-tumor T cells has enabled the identification of tumor antigens and subsequently the generation of cancer vaccines designed to provoke an anti-tumor immune response. Anti-tumor T cells are localized within cancer patients, including in the blood (where they can be found in the peripheral blood mononuclear cell fraction), in primary and secondary lymphoid tissue, *e*.*g*., the spleen, in ascites fluid in ovarian cancer patients (tumor associated lymphocytes or TALs) or within the tumor itself (tumor infiltrating lymphocytes or TILs). Of these, TILs have been the most useful in the identification of tumor antigens and tumor antigen-derived peptides recognized by T cells.

Conventional methods to generate TILs involve mincing tumor biopsy tissue and culturing the cell suspension *in vitro* in the presence of the T cell growth factor interleukin-2 (IL-2). Over a period of several days, the combination of the tumor cells and IL-2 can stimulate the proliferation of tumor specific T cells at the expense of tumor cells. In this way, the T cell population is expanded. The T cells derived from the first expansion are subsequently mixed with either mitomycin C-treated or irradiated tumor cells and cultured *in vitro* with IL2 to promote further proliferation and enrichment of tumor reactive T cells. After several rounds of *in vitro* expansion, a potent anti-tumor T cell population can be recovered and used to identify tumor antigens via conventional but tedious expression cloning methodology. Kawakami Y. et al. (1994) Proc. Natl. Acad. Sci. USA **91(9)**:3515-3519.

This currently employed methodology used to generate tumor specific T cells *in vitro* is unreliable and the antigens identified by this method do not necessarily induce an anti-tumor immune response. Numerous experiments demonstrate that the encounter of antigens by mature T cells often results in the induction of tolerance because of ignorance, anergy or physical deletion. Pardoll (1998) Nature Med. **4(5)**:525-531.

The ability of a particular peptide to function as a T cell epitope requires that it bind effectively to the antigen presenting domain of an MHC molecule and also that it display an appropriate set of amino acids that can be specifically recognized by a T cell receptor molecule. While it is possible to identify natural T cell epitopes derived from antigenic polypeptides, these peptide epitopes do not necessarily represent antigens that are optimized for inducing a particular immune response. In fact, it has been shown that it is possible to improve the effectiveness of natural epitopes by introducing single amino or multiple acids substitutions that alter their sequence (Valmori et al. (2000) J. Immunol **164(2)**:1125-1131). Thus, delivery of carefully optimized synthetic peptide epitopes has the potential to provide an improved method to induce a useful immune response.

The introduction into an animal of an antigen has been widely used for the purposes of modulating the immune response, or lack thereof, to the antigen for a variety of purposes. These include vaccination against pathogens, induction of an immune response to a cancerous cell, reduction of an allergic response, reduction of an immune response to a self antigen that occurs as a result of an autoimmune disorder, reduction of allograft rejection, and induction of an immune response to a self antigen for the purpose of contraception.

In the treatment of cancer, a variety of immunotherapeutic approaches have been taken to generate populations of cytotoxic T lymphocytes which specifically recognize and lyse tumor cells. Many of these approaches depend in part on identifying and characterizing tumor-specific antigens.

More recently, certain pathogen- and tumor-related proteins have been immunologically mimicked with synthetic peptides whose amino acid sequence corresponds to that of an antigenic determinant domain of the pathogen- or tumor-related protein. Despite these advances, peptide immunogens based on native sequences generally perform less than optimally with respect to inducing an immune response. Thus, a need exists for modified synthetic antigenic peptide epitopes with enhanced immunomodulatory properties. This invention satisfies this need and provides related advantages as well.

### DISCLOSURE OF THE INVENTION

The present invention provides novel synthetic therapeutic compounds. These compounds are designed to enhance binding to MHC molecules and to enhance immunoregulatory properties relative to their natural counterparts. The synthetic compounds of the invention are useful to modulate an immune response to the synthetic and naturally occurring compounds.

Further provided are polynucleotides encoding the compounds of the invention, gene delivery vehicles comprising these polynucleotides and host cells comprising these polynucleotides.

In addition, the invention provides the use of the polypeptides of the invention, for the preparation of a medicament for inducing an immune response in a subject and delivering these in the context of an MHC molecule.

The compounds of the invention are also useful to generate antibodies that specifically recognize and bind to these molecules. These antibodies are further useful for immunotherapy when administered to a subject.

The invention also provides immune effector cells raised *in vivo* or *in vitro* in the presence and at the expense of an antigen presenting cell that presents the peptide compositions of the invention in the context of an MHC molecule and the use of these immune effector cells for the preparation of a medicament for immunotherapy in a subject.

Further provided by this invention is a composition comprising at least two immunogenic ligands, wherein said immunogenic ligands are individually characterized by an ability to elicit an immune response against the same native ligand, and wherein said immunogenic ligand is selected from the group consisting of FLTDERMPV (SEQ ID NO:3), FLTDEARSV (SEQ ID NO:5), FLDDEIMRV (SEQ ID NO:7) and FLDDEITFV (SEQ ID NO:9) with or without IIEDDRLPV (SEQ ID NO:11). The ligands can be present in a carrier such as a pharmaceutically acceptable carrier.

Also provided by this invention is a host cell comprising at least two immunogenic ligands, wherein said immunogenic ligands are individually characterized by an ability to elicit an immune response against the same native ligand, and wherein said immunogenic ligand is selected from the group consisting of FLTDERMPV (SEQ ID NO:3), FLTDEARSV (SEQ ID NO:5), and FLDDEIMRV (SEQ ID NO:7), FLDDEITFV (SEQ ID NO:9) with or without IIEDDRLPV (SEQ ID NO:11). In one aspect, the host cell is an antigen presenting cell and the immunogenic ligands are presented on the surface of the cell. In a further aspect, the antigen presenting cell is a dendritic cell. The host cells can be present in a carrier, such as a pharmaceutically acceptable carrier.

Still further provided by this invention is a use of an effective amount of two or more immunogenic ligands, wherein each of said immunogenic ligands is characterized by an ability to elicit an immune response against the same native ligand, and wherein said immunogenic ligand is selected from the group consisting of FLTDERMPV (SEQ ID NO:3), FLTDEARSV (SEQ ID NO:5), FLDDEIMRV (SEQ ID NO:7), and FLDDEITFV (SEQ ID NO:9) with or without IIEDDRLPV (SEQ ID NO:11) for the preparation of a medicament for inducing an immune response in a subject.

### DESCRIPTION OF THE SEQUENCE LISTINGS

SEQ ID NO:1. The complete nucleotide sequence of a cDNA encoding the human cancer antigen protease activated receptor-3 (PAR-3). The coding region extends from nucleotide 1 through nucleotide 3801.
SEQ ID NO:2. The amino acid sequence of the native human cancer antigen PAR-3. The compounds of the invention are variations based on native peptide 700-708.
SEQ ID NO:3. The amino acid sequence of compound 1.
SEQ ID NO:4. The polynucleotide sequence encoding compound 1.
SEQ ID NO:5. The amino acid sequence of compound 2.
SEQ ID NO:6. The polynucleotide sequence encoding compound 2.
SEQ ID NO:7. The amino acid sequence of compound 3.
SEQ ID NO:8. The polynucleotide sequence encoding compound 3.
SEQ ID NO:9. The amino acid sequence of compound 4.
SEQ ID NO:10. The polynucleotide sequence encoding compound 4.
SEQ ID NO:11. The natural epitope of human cancer antigen PAR-3.
SEQ ID NO:12 The polynucleotide sequence encoding the antigen PAR-3.

### MODES OF CARRYING OUT THE INVENTION

The practice of the present invention employs, unless otherwise indicated, conventional techniques of molecular biology (including recombinant techniques), microbiology, cell biology, biochemistry and immunology, which are within the skill of the art. Such techniques are explained fully in the literature. These methods are described in the following publications. See, *e*.*g*., Sambrook et al. MOLECULAR CLONING: A LABORATORY MANUAL, 2^{nd} edition (1989); CURRENT PROTOCOLS IN MOLECULAR BIOLOGY (F. M. Ausubel et al. eds. (1987)); the series METHODS IN ENZYMOLOGY (Academic Press, Inc.); PCR: A PRACTICAL APPROACH (M. MacPherson et al. IRL Press at Oxford University Press (1991)); PCR 2: A PRACTICAL APPROACH (M.J. MacPherson, B.D. Hames and G.R. Taylor eds. (1995)); ANTIBODIES, A LABORATORY MANUAL (Harlow and Lane eds. (1988)); and ANIMAL CELL CULTURE (R.I. Freshney ed. (1987)).

### Definitions

As used herein, certain terms may have the following defined meanings.

As used in the specification and claims, the singular form "a," "an" and "the" include plural references unless the context clearly dictates otherwise. For example, the term "a cell" includes a plurality of cells, including mixtures thereof.

As used herein, the term "comprising" is intended to mean that the compositions and methods include the recited elements, but not excluding others. "Consisting essentially of" when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of" shall mean excluding more than trace elements of other ingredients and substantial method steps for administering the compositions of this invention. Embodiments defined by each of these transition terms are within the scope of this invention.

A "native" or "natural" antigen is a polypeptide, protein or a fragment which contains an epitope, which has been isolated from a natural biological source, and which can specifically bind to an antigen receptor, in particular a T cell antigen receptor (TCR), in a subject.

The term "antigen" is well understood in the art and includes substances which are immunogenic, *i.e*., immunogens, as well as substances which induce immunological unresponsiveness, or anergy, *i.e*., anergens.

An "altered antigen" is one having a primary sequence that is different from that of the corresponding wild-type antigen. Altered antigens can be made by synthetic or recombinant methods and include, but are not limited to, antigenic peptides that are differentially modified during or after translation, *e.g.,* by phosphorylation, glycosylation, cross-linking, acylation, proteolytic cleavage, linkage to an antibody molecule, membrane molecule or other ligand. (Ferguson et al. (1988) Ann. Rev. Biochem. 57:285-320). A synthetic or altered antigen of the invention is intended to bind to the same TCR as the natural epitope.

A "self-antigen" also referred to herein as a native or wild-type antigen is an antigenic peptide that induces little or no immune response in the subj ect due to self-tolerance to the antigen. An example of a self-antigen is the melanoma specific antigen gp100.

The term "tumor associated antigen" or "TAA" refers to an antigen that is associated with or specific to a tumor. Examples of known TAAs include gp100, MART and MAGE.

The terms "major histocompatibility complex" or "MHC" refers to a complex of genes encoding cell-surface molecules that are required for antigen presentation to T cells and for rapid graft rejection. In humans, the MHC is also known as the "human leukocyte antigen" or "HLA" complex. The proteins encoded by the MHC are known as "MHC molecules" and are classified into class I and class II MHC molecules. Class I MHC includes membrane heterodimeric proteins made up of an α chain encoded in the MHC noncovalently linked with the β2-microglobulin. Class I MHC molecules are expressed by nearly all nucleated cells and have been shown to function in antigen presentation to CD8⁺ T cells. Class I molecules include HLA-A, B, and C in humans. Class II MHC molecules also include membrane heterodimeric proteins consisting of noncovalently associated α and β chains. Class II MHC molecules are known to function in CD4⁺ T cells and, in humans, include HLA-DP, -DQ, and DR. In a preferred embodiment, invention compositions and ligands can complex with MHC molecules of any HLA type. Those of skill in the art are familiar with the serotypes and genotypes of the HLA. See: http://bimas.dcrt.nih.gov/cgi-bin/molbio/hla coefficient viewing page. Rammensee H.G., Bachmann J., and Stevanovic S. MHC Ligands and Peptide Motifs (1997) Chapman & Hall Publishers; Schreuder G.M. Th. et al. The HLA dictionary (1999) Tissue Antigens **54**:409-437.

The term "antigen-presenting matrix", as used herein, intends a molecule or molecules which can present antigen in such a way that the antigen can be bound by a T-cell antigen receptor on the surface of a T cell. An antigen-presenting matrix can be on the surface of an antigen-presenting cell (APC), on a vesicle preparation of an APC, or can be in the form of a synthetic matrix on a solid support such as a bead or a plate. An example of a synthetic antigen-presenting matrix is purified MHC class I molecules complexed to β2-microglobulin, multimers of such purified MHC class I molecules, purified MHC Class II molecules, or functional portions thereof, attached to a solid support.

The term "antigen presenting cells (APC)" refers to a class of cells capable of presenting one or more antigens in the form of antigen-MHC complex recognizable by specific effector cells of the immune system, and thereby inducing an effective cellular immune response against the antigen or antigens being presented. While many types of cells may be capable of presenting antigens on their cell surface for T-cell recognition, only professional APCs have the capacity to present antigens in an efficient amount and further to activate T-cells for cytotoxic T-lymphocyte (CTL) responses. APCs can be intact whole cells such as macrophages, B-cells and dendritic cells; or other molecules, naturally occurring or synthetic, such as purified MHC class I molecules complexed to β2-microglobulin.

The term "dendritic cells (DC)" refers to a diverse population of morphologically similar cell types found in a variety of lymphoid and non-lymphoid tissues (Steinman (1991) Ann. Rev. Immunol. **9**:271-296). Dendritic cells constitute the most potent and preferred APCs in the organism. A subset, if not all, of dendritic cells are derived from bone marrow progenitor cells, circulate in small numbers in the peripheral blood and appear either as immature Langerhans' cells or terminally differentiated mature cells. While the dendritic cells can be differentiated from monocytes, they possess distinct phenotypes. For example, a particular differentiating marker, CD14 antigen, is not found in dendritic cells but is possessed by monocytes. Also, mature dendritic cells are not phagocytic, whereas the monocytes are strongly phagocytosing cells. It has been shown that DCs provide all the signals necessary for T cell activation and proliferation.

The term "antigen presenting cell recruitment factors" or "APC recruitment factors" include both intact, whole cells as well as other molecules that are capable of recruiting antigen presenting cells. Examples of suitable APC recruitment factors include molecules such as interleukin 4 (IL4), granulocyte macrophage colony stimulating factor (GM-CSF), Sepragel and macrophage inflammatory protein 3 alpha (MIP3α). These are available from Immunex, Schering-Plough and R&D Systems (Minneapolis, MN). They also can be recombinantly produced using the methods disclosed in CURRENT PROTOCOLS IN MOLECULAR BIOLOGY (F.M. Ausubel et al., eds. (1987)). Peptides, proteins and compounds having the same biological activity as the above-noted factors are included within the scope of this invention.

The term "immune effector cells" refers to cells capable of binding an antigen and which mediate an immune response. These cells include, but are not limited to, T cells, B cells, monocytes, macrophages, NK cells and cytotoxic T lymphocytes (CTLs), for example CTL lines, CTL clones, and CTLs from tumor, inflammatory, or other infiltrates. Certain diseased tissue expresses specific antigens and CTLs specific for these antigens have been identified. For example, approximately 80% of melanomas express the antigen known as GP-100.

The term "immune effector molecule" as used herein, refers to molecules capable of antigen-specific binding, and includes antibodies, T cell antigen receptors, and MHC Class I and Class II molecules.

A "naïve" immune effector cell is an immune effector cell that has never been exposed to an antigen capable of activating that cell. Activation of naïve immune effector cells requires both recognition of the peptide:MHC complex and the simultaneous delivery of a costimulatory signal by a professional APC in order to proliferate and differentiate into antigen-specific armed effector T cells.

"Immune response" broadly refers to the antigen-specific responses of lymphocytes to foreign substances. Any substance that can elicit an immune response is said to be "immunogenic" and is referred to as an "immunogen". All immunogens are antigens, however, not all antigens are immunogenic. An immune response of this invention can be humoral (via antibody activity) or cell-mediated (via T cell activation).

The term "ligand" as used herein refers to any molecule that binds to a specific site on another molecule. In other words, the ligand confers the specificity of the protein in a reaction with an immune effector cell. It is the ligand site within the protein that combines directly with the complementary binding site on the immune effector cell.

In a preferred embodiment, a ligand of the invention binds to an antigenic determinant or epitope on an immune effector cell, such as an antibody or a T cell receptor (TCR). A ligand may be an antigen, peptide, protein or epitope of the invention.

As used herein, the term "educated, antigen-specific immune effector cell", is an immune effector cell as defined above, which has previously encountered an antigen. In contrast with its naive counterpart, activation of an educated, antigen-specific immune effector cell does not require a costimulatory signal. Recognition of the peptide:MHC complex is sufficient.

"Activated", when used in reference to a T cell, implies that the cell is no longer in Go phase, and begins to produce one or more of cytotoxins, cytokines, and other related membrane-associated proteins characteristic of the cell type *(e.g.,* CD8⁺ or CD4⁺), is capable of recognizing and binding any target cell that displays the particular antigen on its surface, and releasing its effector molecules.

In the context of the present invention, the term "recognized" intends that a composition of the invention, comprising one or more ligands, is recognized and bound by an immune effector cell wherein such binding initiates an effective immune response. Assays for determining whether a ligand is recognized by an immune effector cell are known in the art and are described herein.

The term "preferentially recognized" intends that the specificity of a composition or ligand of the invention is restricted to immune effector cells that recognize and bind the native ligand.

The term "cross-reactive" is used to describe compounds of the invention which are functionally overlapping. More particularly, the immunogenic properties of a native ligand and/or immune effector cells activated thereby are shared to a certain extent by the altered ligand such that the altered ligand is "cross-reactive" with the native ligand and/or the immune effector cells activated thereby. For purposes of this invention, cross-reactivity is manifested at multiple levels: (i) at the ligand level, *e.g*., the altered ligands can bind the TCR of and activate native ligand CTLs; (ii) at the T cell level, *i.e.,* altered ligands of the invention bind the TCR of and activate a population of T cells (distinct from the population of native ligand CTLs) which can effectively target and lyse cells displaying the native ligand; and (iii) at the antibody level, *e.g.,* "anti"-altered ligand antibodies can detect, recognize and bind the native ligand and initiate effector mechanisms in an immune response which ultimately result in elimination of the native ligand from the host.

As used herein, the term "inducing an immune response in a subject" is a term well understood in the art and intends that an increase of at least about 2-fold, more preferably at least about 5-fold, more preferably at least about 10-fold, more preferably at least about 100-fold, even more preferably at least about 500-fold, even more preferably at least about 1000-fold or more in an immune response to an antigen (or epitope) can be detected or measured, after introducing the antigen (or epitope) into the subject, relative to the immune response (if any) before introduction of the antigen (or epitope) into the subject. An immune response to an antigen (or epitope), includes, but is not limited to, production of an antigen-specific (or epitope-specific) antibody, and production of an immune cell expressing on its surface a molecule which specifically binds to an antigen (or epitope). Methods of determining whether an immune response to a given antigen (or epitope) has been induced are well known in the art. For example, antigen-specific antibody can be detected using any of a variety of immunoassays known in the art, including, but not limited to, ELISA, wherein, for example, binding of an antibody in a sample to an immobilized antigen (or epitope) is detected with a detectably-labeled second antibody *(e.g.,* enzyme-labeled mouse anti-human Ig antibody).

"Co-stimulatory molecules" are involved in the interaction between receptor-ligand pairs expressed on the surface of antigen presenting cells and T cells. Research accumulated over the past several years has demonstrated convincingly that resting T cells require at least two signals for induction of cytokine gene expression and proliferation (Schwartz R.H. (1990) Science **248**:1349-1356 and Jenkins M.K. (1992) Immunol. Today **13**:69-73). One signal, the one that confers specificity, can be produced by interaction of the TCR/CD3 complex with an appropriate MHC/peptide complex. The second signal is not antigen specific and is termed the "co-stimulatory" signal. This signal was originally defined as an activity provided by bone-marrow-derived accessory cells such as macrophages and dendritic cells, the so called "professional" APCs. Several molecules have been shown to enhance co-stimulatory activity. These are heat stable antigen (HSA) (Liu Y. et al. (1992) J. Exp. Med. **175**:437-445), chondroitin sulfate-modified MHC invariant chain (Ii-CS) (Naujokas M.F. et al. (1993) Cell **74**:257-268), intracellular adhesion molecule 1 (ICAM-1) (Van Seventer G.A. (1990) J. Immunol. **144**:4579-4586), B7-1, and B7-2/B70 (Schwartz R.H. (1992) Cell **71**:1065-1068). These molecules each appear to assist co-stimulation by interacting with their cognate ligands on the T cells. Co-stimulatory molecules mediate co-stimulatory signal(s), which are necessary, under normal physiological conditions, to achieve full activation of naïve T cells. One exemplary receptor-ligand pair is the B7 co-stimulatory molecule on the surface of APCs and its counter-receptor CD28 or CTLA-4 on T cells (Freeman et al. (1993) Science **262**:909-911; Young et al. (1992) J. Clin. Invest. **90**:229 and Nabavi et al. (1992) Nature **360**:266-268). Other important co-stimulatory molecules are CD40, CD54, CD80, and CD86. The term "co-stimulatory molecule" encompasses any single molecule or combination of molecules which, when acting together with a peptide/MHC complex bound by a TCR on the surface of a T cell, provides a co-stimulatory effect which achieves activation of the T cell that binds the peptide. The term thus encompasses B7, or other co-stimulatory molecule(s) on an antigen-presenting matrix such as an APC, fragments thereof (alone, complexed with another molecule(s), or as part of a fusion protein) which, together with peptide/MHC complex, binds to a cognate ligand and results in activation of the T cell when the TCR on the surface of the T cell specifically binds the peptide. Co-stimulatory molecules are commercially available from a variety of sources, including, for example, Beckman Coulter, Inc. (Fullerton, CA). It is intended, although not always explicitly stated, that molecules having similar biological activity as wild-type or purified co-stimulatory molecules (e.g., recombinantly produced or muteins thereof) are intended to be used within the spirit and scope of the invention.

As used herein, "solid phase support" or "solid support", used interchangeably, is not limited to a specific type of support. Rather a large number of supports are available and are known to one of ordinary skill in the art. Solid phase supports include silica gels, resins, derivatized plastic films, glass beads, cotton, plastic beads, alumina gels. As used herein, "solid support" also includes synthetic antigen-presenting matrices, cells, and liposomes. A suitable solid phase support may be selected on the basis of desired end use and suitability for various protocols. For example, for peptide synthesis, solid phase support may refer to resins such as polystyrene *(e.g.,* PAM-resin obtained from Bachem Inc., Peninsula Laboratories, etc.), POLYHIPE® resin (obtained from Aminotech, Canada), polyamide resin (obtained from Peninsula Laboratories), polystyrene resin grafted with polyethylene glycol (TentaGel®, Rapp Polymere, Tubingen, Germany) or polydimethylacrylamide resin (obtained from Milligen/Biosearch, California).

The term "immunomodulatory agent", as used herein, is a molecule, a macromolecular complex, or a cell that modulates an immune response and encompasses a synthetic antigenic peptide of the invention alone or in any of a variety of formulations described herein; a polypeptide comprising a synthetic antigenic peptide of the invention; a polynucleotide encoding a peptide or polypeptide of the invention; a synthetic antigenic peptide of the invention bound to a Class I or a Class II MHC molecule on an antigen-presenting matrix, including an APC and a synthetic antigen-presenting matrix (in the presence or absence of co-stimulatory molecule(s)); a synthetic antigenic peptide of the invention covalently or non-covalently complexed to another molecule(s) or macromolecular structure; and an educated, antigen-specific immune effector cell which is specific for a peptide of the invention.

The term "modulate an immune response" includes inducing (increasing, eliciting) an immune response; and reducing (suppressing) an immune response. An immunomodulatory method (or protocol) is one that modulates an immune response in a subject.

As used herein, the term "cytokine" refers to any one of the numerous factors that exert a variety of effects on cells, for example, inducing growth or proliferation. Nonlimiting examples of cytokines which may be used alone or in combination in the practice of the present invention include, interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-1α), interleukin-11 (IL-11), MIP-11, leukemia inhibitory factor (LIF), c-kit ligand, thrombopoietin (TPO) and flt3 ligand. The present invention also includes culture conditions in which one or more cytokine is specifically excluded from the medium. Cytokines are commercially available from several vendors such as, for example, Genzyme (Framingham, MA), Genentech (South San Francisco, CA), Amgen (Thousand Oaks, CA), R&D Systems (Minneapolis, MN) and Immunex (Seattle, WA). It is intended, although *not* always explicitly stated, that molecules having similar biological activity as wild-type or purified cytokines (*e*.*g*., recombinantly produced or muteins thereof) are intended to be used within the spirit and scope of the invention.

The terms "polynucleotide" and "nucleic acid molecule" are used interchangeably to refer to polymeric forms of nucleotides of any length. The polynucleotides may contain deoxyribonucleotides, and/or ribonucleotides. The term "polynucleotide" includes, for example, single-stranded, doublestranded and triple helical molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers.

The term "peptide" is used in its broadest sense to refer to a compound of two or more subunit amino acids. The subunits are linked by peptide bonds. As used herein the term "amino acid" refers to either natural or synthetic amino acids, includes glycine and both the D or L optical isomers. A peptide of three or more amino acids is commonly called an oligopeptide if the peptide chain is short. If the peptide chain is long, the peptide is commonly called a polypeptide or a protein.

The term "genetically modified" means containing and/or expressing a foreign gene or nucleic acid sequence which in turn, modifies the genotype or phenotype of the cell or its progeny. In other words, it refers to any addition, deletion or disruption to a cell's endogenous nucleotides.

As used herein, "expression" refers to the process by which polynucleotides are transcribed into mRNA and translated into peptides, polypeptides, or proteins. If the polynucleotide is derived from genomic DNA, expression may include splicing of the mRNA, if an appropriate eukaryotic host is selected. Regulatory elements required for expression include promoter sequences to bind RNA polymerase and transcription initiation sequences for ribosome binding. For example, a bacterial expression vector includes a promoter such as the *lac* promoter and for transcription initiation the Shine-Dalgarno sequence and the start codon AUG (Sambrook et al. (1989) *supra).* Similarly, an eukaryotic expression vector includes a heterologous or homologous promoter for RNA polymerase II, a downstream polyadenylation signal, the start codon AUG, and a termination codon for detachment of the ribosome. Such vectors can be obtained commercially or assembled by the sequences described in methods well known in the art, for example, the methods described below for constructing vectors in general.

"Under transcriptional control" is a term well understood in the art and indicates that transcription of a polynucleotide sequence, usually a DNA sequence, depends on its being operatively linked to an element which contributes to the initiation of, or promotes, transcription. "Operatively linked" refers to a juxtaposition wherein the elements are in an arrangement allowing them to function.

A "gene delivery vehicle" is defined as any molecule that can carry inserted polynucleotides into a host cell. Examples of gene delivery vehicles are liposomes, biocompatible polymers, including natural polymers and synthetic polymers; lipoproteins; polypeptides; polysaccharides; lipopolysaccharides; artificial viral envelopes; metal particles; and bacteria, or viruses, such as baculovirus, adenovirus and retrovirus, bacteriophage, cosmid, plasmid, fungal vectors and other recombination vehicles typically used in the art which have been described for expression in a variety of eukaryotic and prokaryotic hosts, and may be used for gene therapy as well as for simple protein expression.

"Gene delivery," "gene transfer," and the like as used herein, are terms referring to the introduction of an exogenous polynucleotide (sometimes referred to as a "transgene") into a host cell, irrespective of the method used for the introduction. Such methods include a variety of well-known techniques such as vector-mediated gene transfer (by, *e.g.,* viral infection/transfection, or various other protein-based or lipid-based gene delivery complexes) as well as techniques facilitating the delivery of "naked" polynucleotides (such as electroporation, "gene gun" delivery and various other techniques used for the introduction of polynucleotides). The introduced polynucleotide may be stably or transiently maintained in the host cell. Stable maintenance typically requires that the introduced polynucleotide either contains an origin of replication compatible with the host cell or integrates into a replicon of the host cell such as an extrachromosomal replicon (*e.g*., a plasmid) or a nuclear or mitochondrial chromosome. A number of vectors are known to be capable of mediating transfer of genes to mammalian cells, as is known in the art and described herein.

A "viral vector" is defined as a recombinantly produced virus or viral particle that comprises a polynucleotide to be delivered into a host cell, either *in vivo, ex vivo* or *in vitro*. Examples of viral vectors include retroviral vectors, adenovirus vectors, adeno-associated virus vectors, alphavirus vectors and the like. Alphavirus vectors, such as Semliki Forest virus-based vectors and Sindbis virus-based vectors, have also been developed for use in gene therapy and immunotherapy. See, Schlesinger and Dubensky (1999) Curr Opin Biotechnol. **5**:434-439 and Ying et al. (1999) Nat. Med. **5**(7):823-827. In aspects where gene transfer is mediated by a retroviral vector, a vector construct refers to the polynucleotide comprising the retroviral genome or part thereof, and a therapeutic gene. As used herein, "retroviral mediated gene transfer" or "retroviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, retroviral vector refers to a viral particle capable of introducing exogenous nucleic acid into a cell through a viral or viral-like entry mechanism.

Retroviruses carry their genetic information in the form of RNA; however, once the virus infects a cell, the RNA is reverse-transcribed into the DNA form which integrates into the genomic DNA of the infected cell. The integrated DNA form is called a provirus.

In aspects where gene transfer is mediated by a DNA viral vector, such as an adenovirus (Ad) or adeno-associated virus (AAV), a vector construct refers to the polynucleotide comprising the viral genome or part thereof, and a transgene. Adenoviruses (Ads) are a relatively well characterized, homogenous group of viruses, including over 50 serotypes. See, *e.g.,* WO 95/27071. Ads are easy to grow and do not require integration into the host cell genome. Recombinant Ad-derived vectors, particularly those that reduce the potential for recombination and generation of wild-type virus, have also been constructed. See, WO 95/00655 and WO 95/11984. Wild-type AAV has high infectivity and specificity integrating into the host cell's genome. See, Hermonat and Muzyczka (1984) Proc. Natl. Acad. Sci. USA **81**:6466-6470 and Lebkowski et al. (1988) Mol. Cell. Biol. **8**:3988-3996.

Vectors that contain both a promoter and a cloning site into which a polynucleotide can be operatively linked are well known in the art. Such vectors are capable of transcribing RNA *in vitro* or *in vivo,* and are commercially available from sources such as Stratagene (La Jolla, CA) and Promega Biotech (Madison, WI). In order to optimize expression and/or *in vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

Gene delivery vehicles also include several non-viral vectors, including DNA/liposome complexes, and targeted viral protein-DNA complexes. Liposomes that also comprise a targeting antibody or fragment thereof can be used in the methods of this invention. To enhance delivery to a cell, the nucleic acid or proteins of this invention can be conjugated to antibodies or binding fragments thereof which bind cell surface antigens, e.g., TCR, CD3 or CD4.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Examples of stringent hybridization conditions include: incubation temperatures of about 25°C to about 37°C; hybridization buffer concentrations of about 6 X SSC to about 10 X SSC; formamide concentrations of about 0% to about 25%; and wash solutions of about 6 X SSC. Examples of moderate hybridization conditions include: incubation temperatures of about 40°C to about 50°C; buffer concentrations of about 9 X SSC to about 2 X SSC; formamide concentrations of about 30% to about 50%; and wash solutions of about 5 X SSC to about 2 X SSC. Examples of high stringency conditions include: incubation temperatures of about 55°C to about 68°C; buffer concentrations of about 1 X SSC to about 0.1 X SSC; formamide concentrations of about 55% to about 75%; and wash solutions of about 1 X SSC, 0.1 X SSC, or deionized water. In general, hybridization incubation times are from 5 minutes to 24 hours, with 1, 2, or more washing steps, and wash incubation times are about 1, 2, or 15 minutes. SSC is 0.15 M NaCl and 15 mM citrate buffer. It is understood that equivalents of SSC using other buffer systems can be employed.

"*In vivo"* gene delivery, gene transfer, gene therapy and the like as used herein, are terms referring to the introduction of a vector comprising an exogenous polynucleotide directly into the body of an organism, such as a human or non-human mammal, whereby the exogenous polynucleotide is introduced to a cell of such organism *in vivo*.

The term "isolated" means separated from constituents, cellular and otherwise, in which the polynucleotide, peptide, polypeptide, protein, antibody, or fragments thereof, are normally associated with in nature. For example, with respect to a polynucleotide, an isolated polynucleotide is one that is separated from the 5' and 3' sequences with which it is normally associated in the chromosome. As is apparent to those of skill in the art, a non-naturally occurring polynucleotide, peptide, polypeptide, protein, antibody, or fragments thereof, does not require "isolation" to distinguish it from its naturally occurring counterpart. In addition, a "concentrated", "separated" or "diluted" polynucleotide, peptide, polypeptide, protein, antibody, or fragments thereof, is distinguishable from its naturally occurring counterpart in that the concentration or number of molecules per volume is greater than "concentrated" or less than "separated" than that of its naturally occurring counterpart. A polynucleotide, peptide, polypeptide, protein, antibody, or fragments thereof, which differs from the naturally occurring counterpart in its primary sequence or for example, by its glycosylation pattern, need not be present in its isolated form since it is distinguishable from its naturally occurring counterpart by its primary sequence, or alternatively, by another characteristic such as glycosylation pattern. Although not explicitly stated for each of the inventions disclosed herein, it is to be understood that all of the above embodiments for each of the compositions disclosed below and under the appropriate conditions, are provided by this invention. Thus, a non-naturally occurring polynucleotide is provided as a separate embodiment from the isolated naturally occurring polynucleotide. A protein produced in a bacterial cell is provided as a separate embodiment from the naturally occurring protein isolated from a eucaryotic cell in which it is produced in nature.

"Host cell," "target cell" or "recipient cell" are intended to include any individual cell or cell culture which can be or have been recipients for vectors or the incorporation of exogenous nucleic acid molecules, polynucleotides and/or proteins. It also is intended to include progeny of a single cell, and the progeny may not necessarily be completely identical (in morphology or in genomic or total DNA complement) to the original parent cell due to natural, accidental, or deliberate mutation. The cells may be procaryotic or eucaryotic, and include but are not limited to bacterial cells, yeast cells, animal cells, and mammalian cells, *e.g*., murine, rat, simian or human.

A "subject" is a vertebrate, preferably a mammal, more preferably a human. Mammals include, but are not limited to, murines, simians, humans, farm animals, sport animals, and pets.

A "control" is an alternative subject or sample used in an experiment for comparison purpose. A control can be "positive" or "negative". For example, where the purpose of the experiment is to determine a correlation of an altered expression level of a gene with a particular type of cancer, it is generally preferable to use a positive control (a subject or a sample from a subject, carrying such alteration and exhibiting syndromes characteristic of that disease), and a negative control (a subject or a sample from a subject lacking the altered expression and clinical syndrome of that disease).

The terms "cancer," "neoplasm," and "tumor," used interchangeably and in either the singular or plural form, refer to cells that have undergone a malignant transformation that makes them pathological to the host organism. Primary cancer cells (that is, cells obtained from near the site of malignant transformation) can be readily distinguished from non-cancerous cells by well-established techniques, particularly histological examination. The definition of a cancer cell, as used herein, includes not only a primary cancer cell, but also any cell derived from a cancer cell ancestor. This includes metastasized cancer cells, and *in vitro* cultures and cell lines derived from cancer cells. When referring to a type of cancer that normally manifests as a solid tumor, a "clinically detectable" tumor is one that is detectable on the basis of tumor mass; *e.g.*, by such procedures as CAT scan, magnetic resonance imaging (MRI), X-ray, ultrasound or palpation. Biochemical or immunologic findings alone may be insufficient to meet this definition.

"Suppressing" tumor growth indicates a growth state that is curtailed compared to growth without contact with educated, antigen-specific immune effector cells described herein. Tumor cell growth can be assessed by any means known in the art, including, but not limited to, measuring tumor size, determining whether tumor cells are proliferating using a ³H-thymidine incorporation assay, or counting tumor cells. "Suppressing" tumor cell growth means any or all of the following states: slowing, delaying, and "suppressing" tumor growth indicates a growth state that is curtailed when stopping tumor growth, as well as tumor shrinkage.

The term "culturing" refers to the *in vitro* propagation of cells or organisms on or in media of various kinds. It is understood that the descendants of a cell grown in culture may not be completely identical (morphologically, genetically, or phenotypically) to the parent cell. By "expanded" is meant any proliferation or division of cells.

A "composition" is intended to mean a combination of active agent and another compound or composition, inert (for example, a detectable agent or label) or active, such as an adjuvant.

A "pharmaceutical composition" is intended to include the combination of an active agent with a carrier, inert or active, making the composition suitable for diagnostic or therapeutic use *in vitro, in vivo* or *ex vivo*.

As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin REMINGTON'S PHARM. SCI., 15th Ed. (Mack Publ. Co., Easton (1975)).

An "effective amount" is an amount sufficient to effect beneficial or desired results. An effective amount can be administered in one or more administrations, applications or dosages. The present invention provides compounds having the following structures:

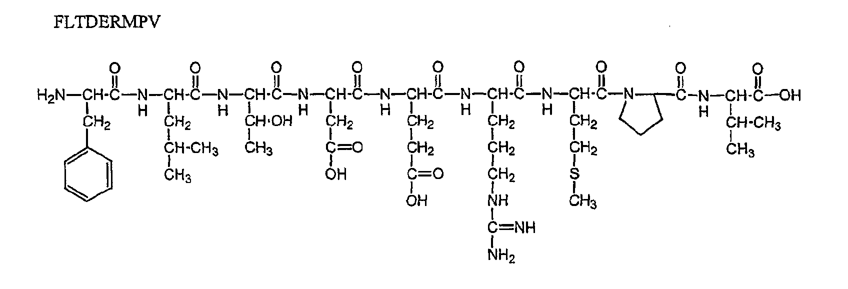

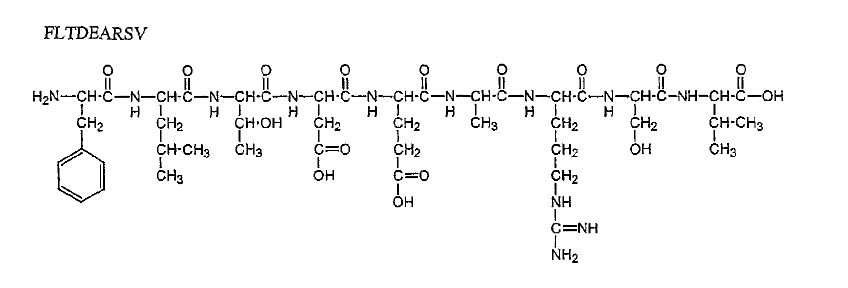

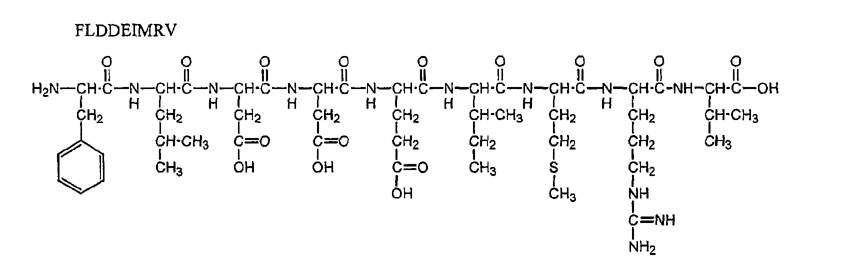

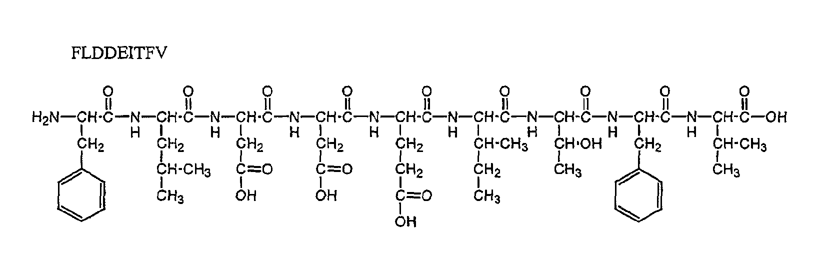

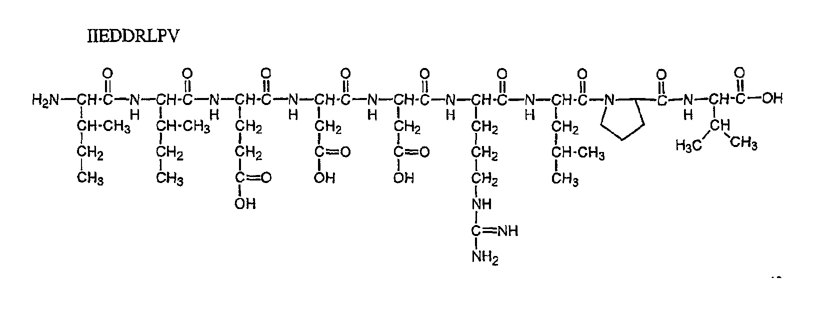

The present invention also provides compositions that exhibit enhancing binding to MHC molecules and are cross-reactive with and useful for modulating immune responses to the cognate native ligands and their corresponding native proteins.

This invention further provides compositions which are useful as components of anti-cancer vaccines and to expand immune effector cells that are specific for cancers characterized by expression of the human cancer antigen Partition -3(PAR-3). An example of this type of cancer is ovarian cancer.

PAR-3 protein has been described as belonging to PAR (partitioning-defective) proteins which are essential for asymmetric cell division and polarized growth. PAR-3 is a large protein with three PDZ domains which mediate protein-protein interactions (Jobertly G et al, (2000) Nature Cell Biology 2, 531-535, Database NCBI, accession no. AF252293).

WO 00/43511 describes CASB612 polypeptides and polynucleotides and methods for producing such polypeptides by recombinant techniques. Methods for utilizing CASB12 polypeptides and polynucleotides in diagnostics, and vaccines for prophylactic and therapeutic treatment of cancers, particularly ovarian and colon cancers, autoimmune diseases, and related conditions are also disclosed.

In one embodiment, the altered ligands of the invention have comparable affinity for MHC binding as the native ligand. It has been demonstrated that peptide:MHC class I binding properties correlate with immunogenicity (Sette A. et al. (1994) J. Immunol. 153(12):5586-5592; van der Burg S.H. et al. (1996) J. Immunol. 156:3308-3314). In a preferred embodiment, altered ligands of the invention bind to a TCR with a higher affinity than of that the "natural" ligand. Comparative binding of the native and altered ligands of the invention to an MHC class I molecule can be measured by methods that are known in the art and include, but are not limited to, calculating the affinity based on an algorithm (see, for example, Parker et al. (1992) J. Immunol. 149:3580-3587) and experimentally determining binding affinity (see, for example, Tan et al. (1997) J. Immunol. Meth. 209(1):25-36). For example, the relative binding of a peptide to a class I molecule can be measured on the basis of binding of a radiolabeled standard peptide to detergent-solubilized MHC molecules, using various concentrations of test peptides *(e.g.,* ranging from 100 mM to 1nM). MHC class I heavy chain and β2-microglobulin are coincubated with a fixed concentration (*e*.*g*., 5 nM) radiolabeled standard (control) peptide and various concentrations of a test peptide for a suitable period of time *(e.g.,* 2 hours to 72 hours) at room temperature in the presence of a mixture of protease inhibitors. A control tube contains standard peptide and MHC molecules, but no test peptide. The percent MHC-bound radioactivity is determined by gel filtration. The IC₅₀ (concentration of test peptide which results in 50% inhibition of binding of control peptide) is calculated for each peptide. Additional methods for determining binding affinity to a TCR are known in the art and include, but are not limited to, those described in al-Ramadi et al. (1992) J. Immunol. 155(2):662-673; and Zuegel et al. (1998) J. Immunol. **161**(4):1705-1709.

In another embodiment, the altered ligands of the invention elicit comparable antigen-specific T cell activation relative to their native ligand counterpart. In a preferred embodiment, altered ligands of the invention elicit a stronger antigen-specific T cell activation relative to their native ligand counterpart. Methods for determining immunogenicity of invention ligands are known in the art and are further described herein.

In one embodiment, compositions of the invention comprise two or more immunogenic ligands of the invention. In one aspect, such compositions may comprise two or more copies of a single ligand. In another aspect, such compositions may comprise two or more ligands, wherein each ligand of said two or more ligands is distinct from all other ligands in said composition. In one embodiment, the two or more immunogenic ligands are covalently linked.

The present invention also provides novel synthetic antigenic peptides designed for enhancing binding to MHC molecules and useful for modulating immune responses to the synthetic peptide epitope and the corresponding native peptides from which they are derived. The synthetic antigenic peptide epitope sequences of the present invention differ from their natural counterparts in that they contain alterations in amino acid sequence, relative to the native sequence, in the MHC Class I binding domain which is designed to confer tighter binding to the MHC. They further contain mutations in the putative T cell receptor-binding domain designed to increase affinity for the T cell antigen receptor. These differences from the native sequence are designed to confer advantages in the methods of the present invention over the native sequence, in that the synthetic antigenic peptide epitopes of the invention will have enhanced immunomodulatory properties.

This invention provides novel, synthetic antigenic peptide sequences, which are useful as components of anti-cancer vaccines and to expand immune effector cells that are specific for cancers characterized by expression of the human cancer antigen PAR-3. The peptides, differ from the natural epitope in that they contain mutations in the putative HLA-A2 binding domain (amino acids 1, 2 and 9) and T cell receptor binding (TCR) domain (amino acid residues 3-8) conferring tighter binding to the MHC and TCR respectively. Binding of synthetic antigenic peptide of the invention to an MHC Class I molecule can be measured by methods that are known in the art and include, but are not limited to, calculating the affinity based on an algorithm (see, for example, Parker et al. (1992) J. Immunol. **149**:3580-3587); and experimentally determining binding affinity (see, for example, Tan et al. (1997) J. Immunol. Meth. **209(1)**:25-36). For example, the relative binding of a peptide to a Class I molecule can be measured on the basis of binding of a radiolabeled standard peptide to detergent-solubilized MHC molecules, using various concentrations of test peptides *(e.g.,* ranging from 100 mM to 1nM). MHC Class I heavy chain and β2-microglobulin are coincubated with a fixed concentration (*e*.*g*., 5 nM) radiolabeled standard (control) peptide and various concentrations of a test peptide for a suitable period of time (*e*.*g*., 2 hours to 72 hours) at room temperature in the presence of a mixture of protease inhibitors. A control tube contains standard peptide and MHC molecules, but no test peptide. The percent MHC-bound radioactivity is determined by gel filtration. The IC₅₀ (concentration of test peptide which results in 50% inhibition of binding of control peptide) is calculated for each peptide.

Synthetic peptides of the invention are designed to bind to a TCR with a higher affinity than of that the "natural" sequence. Methods for determining binding affinity to a TCR are known in the art and include, but are not limited to, those described in al-Ramadi et al. (1992) J. Immunol. **155**(2):662-673; and Zuegel et al. (1998) J. Immunol. **161**(4):1705-1709.

Further encompassed by the term "synthetic antigenic peptide" are multimers (concatemers) of a synthetic antigenic peptide of the invention, optionally including intervening amino acid sequences as well as polypeptides comprising the sequences and (SEQ ID NO: 3, 5, 7, 9 and 11). The invention also provides polypeptides comprising these sequences wherein the polypeptides are preferentially recognized by human cancer antigen PAR-3 cytotoxic T lymphocytes.

Polypeptides comprising the peptide sequences of the invention can be prepared by altering the sequence of polynucleotides that encode the native human cancer antigen PAR-3 polypeptide sequence. This is accomplished by methods of recombinant DNA technology well know to those skilled in the art. For example, site directed mutagenesis may be performed on recombinant polynucleotides encoding the native huan cancer antigen PAR-3 sequence to introduce changes in the polynucleotide sequence so that the altered polynucleotide encodes the peptides of the invention.

The proteins and polypeptides of this invention can be obtained by chemical synthesis using a commercially available automated peptide synthesizer such as those manufactured by Perkin Elmer/Applied Biosystems, Inc., Model 430A or 431A, Foster City, CA, USA. The synthesized protein or polypeptide can be precipitated and further purified, for example by high performance liquid chromatography (HPLC). Accordingly, this invention also provides a process for chemically synthesizing the proteins of this invention by providing the sequence of the protein and reagents, such as amino acids and enzymes and linking together the amino acids in the proper orientation and linear sequence.

Alternatively, the proteins and polypeptides can be obtained by well-known recombinant methods as described herein using the host cell and vector systems described below.

### Peptide analogues

It is well know to those skilled in the art that modifications can be made to the peptides of the invention to provide them with altered properties. As used herein the term "amino acid" refers to either natural or synthetic amino acids, including glycine and both the D or L optical isomers. A peptide of three or more amino acids is commonly called an oligopeptide if the peptide chain is short. If the peptide chain is long, the peptide is commonly called a polypeptide or a protein.

A synthetic antigenic peptide epitope of the invention can be used in a variety of formulations, which may vary depending on the intended use.

A synthetic antigenic peptide epitope of the invention can be covalently or non-covalently linked (complexed) to various other molecules, the nature of which may vary depending on the particular purpose. For example, a peptide of the invention can be covalently or non-covalently complexed to a macromolecular carrier, including, but not limited to, natural and synthetic polymers, proteins, polysaccharides, polypeptides (amino acids), polyvinyl alcohol, polyvinyl pyrrolidone, and lipids. A peptide can be conjugated to a fatty acid, for introduction into a liposome. U.S. Patent No. 5,837,249. A synthetic peptide of the invention can be complexed covalently or non-covalently with a solid support, a variety of which are known in the art. A synthetic antigenic peptide epitope of the invention can be associated with an antigen-presenting matrix with or without co-stimulatory molecules, as described in more detail below.

Examples of protein carriers include, but are not limited to, superantigens, serum albumin, tetanus toxoid, ovalbumin, thyroglobulin, myoglobulin, and immunoglobulin.

Peptide-protein carrier polymers may be formed using conventional cross-linking agents such as carbodimides. Examples of carbodimides are 1-cyclohexyl-3-(2-morpholinyl-(4-ethyl) carbodiimide (CMC), 1-ethyl-3-(3-dimethyaminopropyl) carbodiimide (EDC) and 1-ethyl-3-(4-azonia-44-dimethylpentyl) carbodiimide.

Examples of other suitable cross-linking agents are cyanogen bromide, glutaraldehyde and succinic anhydride. In general, any of a number of homo-bifunctional agents including a homo-bifunctional aldehyde, a homo-bifunctional epoxide, a homo-bifunctional imido-ester, a homo-bifunctional N-hydroxysuccinimide ester, a homo-bifunctional maleimide, a homo-bifunctional alkyl halide, a homo-bifunctional pyridyl disulfide, a homo-bifunctional aryl halide, a homo-bifunctional hydrazide, a homo-bifunctional diazonium derivative and a homo-bifunctional photoreactive compound may be used. Also included are hetero-bifunctional compounds, for example, compounds having an amine-reactive and a sulfhydryl-reactive group, compounds with an amine-reactive and a photoreactive group and compounds with a carbonyl-reactive and a sulfhydryl-reactive group.

Specific examples of such homo-bifunctional cross-linking agents include the bifunctional N-hydroxysuccinimide esters dithiobis(succinimidylpropionate), disuccinimidyl suberate, and disuccinimidyl tartarate; the bifunctional imido-esters dimethyl adipimidate, dimethyl pimelimidate, and dimethyl suberimidate; the bifunctional sulfhydryl-reactive crosslinkers 1,4-di-[3'-(2'-pyridyldithio) propionamido]butane, bismaleimidohexane, and bis-N-maleimido-1, 8-octane; the bifunctional aryl halides 1,5-difluoro-2,4-dinitrobenzene and 4,4'-difluoro-3,3'-dinitrophenylsulfone; bifunctional photoreactive agents such as bis-[b-(4-azidosalicylamido)ethyl]disulfide; the bifunctional aldehydes formaldehyde, malondialdehyde, succinaldehyde, glutaraldehyde, and adipaldehyde; a bifunctional epoxide such as 1,4-butaneodiol diglycidyl ether; the bifunctional hydrazides adipic acid dihydrazide, carbohydrazide, and succinic acid dihydrazide; the bifunctional diazoniums o-tolidine, diazotized and bis-diazotized benzidine; the bifunctional alkylhalides N1N'-ethylene-bis(iodoacetamide), N1N'-hexamethylene-bis(iodoacetamide), N1N'-undecamethylene-bis(iodoacetamide), as well as benzylhalides and halomustards, such as a1a'-diiodo-p-xylene sulfonic acid and tri(2-chloroethyl)amine, respectively.

Examples of common hetero-bifunctional cross-linking agents that may be used to effect the conjugation of proteins to peptides include, but are not limited to, SMCC (succinimidyl-4-(N-maleimidomethyl)cyclohexane-1-carboxylate), MBS (m-maleimidobenzoyl-N-hydroxysuccinimide ester), SIAB (N-succinimidyl(4-iodoacteyl)aminobenzoate), SMPB (succinimidyl-4-(p-maleimidophenyl)butyrate), GMBS (N-(γ-maleimidobutyryloxy)succinimide ester), MPBH (4-(4-N-maleimidopohenyl) butyric acid hydrazide), M2C2H (4-(N-maleimidomethyl) cyclohexane-1-carboxyl-hydrazide), SMPT (succinimidyloxycarbonyl-á-methyl-á-(2-pyridyldithio)toluene), and SPDP (N-succinimidyl 3-(2-pyridyldithio)propionate).

Cross-linking may be accomplished by coupling a carbonyl group to an amine group or to a hydrazide group by reductive amination.

Peptides of the invention also may be formulated as non-covalent attachment of monomers through ionic, adsorptive, or biospecific interactions. Complexes of peptides with highly positively or negatively charged molecules may be done through salt bridge formation under low ionic strength environments, such as in deionized water. Large complexes can be created using charged polymers such as poly-(L-glutamic acid) or poly-(L-lysine) which contain numerous negative and positive charges, respectively. Adsorption of peptides may be done to surfaces such as microparticle latex beads or to other hydrophobic polymers, forming non-covalently associated peptide-superantigen complexes effectively mimicking cross-linked or chemically polymerized protein. Finally, peptides maybe non-covalently linked through the use ofbiospecific interactions between other molecules. For instance, utilization of the strong affinity of biotin for proteins such as avidin or streptavidin or their derivatives could be used to form peptide complexes. These biotin-binding proteins contain four binding sites that can interact with biotin in solution or be covalently attached to another molecule. Wilchek (1988) Anal. Biochem. **171**:1-32. Peptides can be modified to possess biotin groups using common biotinylation reagents such as the N-hydroxysuccinimidyl ester of D-biotin (NHS-biotin) which reacts with available amine groups on the protein. Biotinylated peptides then can be incubated with avidin or streptavidin to create large complexes. The molecular mass of such polymers can be regulated through careful control of the molar ratio of biotinylated peptide to avidin or streptavidin.

Also provided by this application are the peptides and polypeptides described herein conjugated to a detectable agent for use in the diagnostic methods. For example, detectably labeled peptides and polypeptides can be bound to a column and used for the detection and purification of antibodies. They also are useful as immunogens for the production of antibodies, as described below.

The peptides of this invention also can be combined with various liquid phase carriers, such as sterile or aqueous solutions, pharmaceutically acceptable carriers, suspensions and emulsions. Examples of non-aqueous solvents include propyl ethylene glycol, polyethylene glycol and vegetable oils. When used to prepare antibodies, the carriers also can include an adjuvant that is useful to non-specifically augment a specific immune response. A skilled artisan can easily determine whether an adjuvant is required and select one. However, for the purpose of illustration only, suitable adjuvants include, but are not limited to, Freund's Complete and Incomplete, mineral salts and polynucleotides.

This invention further provides polynucleotides encoding polypeptides comprising the sequences (SEQ ID NOS: 3, 5, 7, or 9) and the complements of these polynucleotides. As used herein, the term "polynucleotide" encompasses DNA, RNA and nucleic acid mimetics. In addition to the sequences identified above or their complements, this invention also provides the anti-sense polynucleotide stand, *e.g.* antisense RNA to these sequences or their complements. One can obtain an antisense RNA using the sequences provided in Seq. ID Nos. 4, 6, 8, 10 and 12, and the methodology described in Van der Krol, et al. (1988) BioTechniques 6:958.

The polynucleotides of this invention can be replicated using PCR. PCR technology is the subject matter of United States Patent Nos. 4,683,195; 4,800,159; 4,754,065; and 4,683,202 and described in PCR: THE POLYMERASE CHAIN REACTION (Mullis et al. eds, Birkhauser Press, Boston (1994)) and references cited therein.

Alternatively, one of skill in the art can use the sequences provided herein and a commercial DNA synthesizer to replicate the DNA. Accordingly, this invention also provides a process for obtaining the polynucleotides of this invention by providing the linear sequence of the polynucleotide, appropriate primer molecules, chemicals such as enzymes and instructions for their replication and chemically replicating or linking the nucleotides in the proper orientation to obtain the polynucleotides. In a separate embodiment, these polynucleotides are further isolated. Still further, one of skill in the art can insert the polynucleotide into a suitable replication vector and insert the vector into a suitable host cell (procaryotic or eucaryotic) for replication and amplification. The DNA so amplified can be isolated from the cell by methods well known to those of skill in the art. A process for obtaining polynucleotides by this method is further provided herein as well as the polynucleotides so obtained.

RNA can be obtained by first inserting a DNA polynucleotide into a suitable host cell. The DNA can be inserted by any appropriate method, *e.g*., by the use of an appropriate gene delivery vehicle (*e.g*., liposome, plasmid or vector) or by electroporation. When the cell replicates and the DNA is transcribed into RNA; the RNA can then be isolated using methods well known to those of skill in the art, for example, as set forth in Sambrook et al. (1989) *supra*. For instance, mRNA can be isolated using various lytic enzymes or chemical solutions according to the procedures set forth in Sambrook, et al. (1989) *supra* or extracted by nucleic-acid-binding resins following the accompanying instructions provided by manufactures.

Polynucleotides exhibiting sequence homology to the sequences encoding the amino acids shown in SEQ ID NOS. 3, 5, 7, 9 and 11, (SEQ ID NOS. 2, 4, 6, 8, 10 or 12) find utility as hybridization probes.

These probes can be used in radioassays (*e*.*g*. Southern and Northern blot analysis) to detect or monitor various cells or tissue containing these cells. The probes also can be attached to a solid support or an array such as a chip for use in high throughput screening assays for the detection of expression of the gene corresponding to one or more polynucleotide(s) of this invention. Accordingly, this invention also provides at least one probe identified as SEQ ID NOS. 4, 6, 8, 10 or 12, or the complement of one of these sequences, attached to a solid support for use in high throughput screens.

The polynucleotides of the present invention also can serve as primers for the detection of genes or gene transcripts that are expressed in APC, for example, to confirm transduction of the polynucleotides into host cells. In this context, amplification means any method employing a primer-dependent polymerase capable of replicating a target sequence with reasonable fidelity. Amplification may be carried out by natural or recombinant DNA-polymerases such as T7 DNA polymerase, Klenow fragment of *E. coli* DNA polymerase, and reverse transcriptase. A preferred length of the primer is the same as that identified for probes, above.

The invention further provides the isolated polynucleotide operatively linked to a promoter of RNA transcription, as well as other regulatory sequences for replication and/or transient or stable expression of the DNA or RNA. As used herein, the term "operatively linked" means positioned in such a manner that the promoter will direct transcription of RNA off the DNA molecule. Examples of such promoters are SP6, T4 and T7. In certain embodiments, cell-specific promoters are used for cell-specific expression of the inserted polynucleotide. Vectors which contain a promoter or a promoter/enhancer, with termination codons and selectable marker sequences, as well as a cloning site into which an inserted piece of DNA can be operatively linked to that promoter are well known in the art and commercially available. For general methodology and cloning strategies, see GENE EXPRESSION TECHNOLOGY (Goeddel ed., Academic Press, Inc. (1991)) and references cited therein and VECTORS: ESSENTIAL DATA SERIES (Gacesa and Ramji, eds., John Wiley & Sons, N.Y. (1994)), which contains maps, functional properties, commercial suppliers and a reference to GenEMBL accession numbers for various suitable vectors. Preferable, these vectors are capable of transcribing RNA *in vitro* or *in vivo.*

Expression vectors containing these nucleic acids are useful to obtain host vector systems to produce proteins and polypeptides. It is implied that these expression vectors must be replicable in the host organisms either as episomes or as an integral part of the chromosomal DNA. Suitable expression vectors include plasmids, viral vectors, including adenoviruses, adeno-associated viruses, retroviruses, cosmids, etc. Adenoviral vectors are particularly useful for introducing genes into tissues *in vivo* because of their high levels of expression and efficient transformation of cells both *in vitro* and *in vivo.* When a nucleic acid is inserted into a suitable host cell, *e.g.,* a procaryotic or a eucaryotic cell and the host cell replicates, the protein can be recombinantly produced. Suitable host cells will depend on the vector and can include mammalian cells, animal cells, human cells, simian cells, insect cells, yeast cells, and bacterial cells constructed using well known methods. See Sambrook, et al. (1989) *supra*. In addition to the use of viral vector for insertion of exogenous nucleic acid into cells, the nucleic acid can be inserted into the host cell by methods well known in the art such as transformation for bacterial cells; transfection using calcium phosphate precipitation for mammalian cells; DEAE-dextran; electroporation; or microinjection. See Sambrook et al. (1989) *supra* for this methodology. Thus, this invention also provides a host cell, *e.g.* a mammalian cell, an animal cell (rat or mouse), a human cell, or a procaryotic cell such as a bacterial cell, containing a polynucleotide encoding a protein or polypeptide or antibody.

The present invention also provides delivery vehicles suitable for delivery of a polynucleotide of the invention into cells (whether *in vivo, ex vivo,* or *in vitro*). A polynucleotide of the invention can be contained within a cloning or expression vector. These vectors (especially expression vectors) can in turn be manipulated to assume any of a number of forms which may, for example, facilitate delivery to and/or entry into a cell.

When the vectors are used for gene therapy *in vivo* or *ex vivo,* a pharmaceutically acceptable vector is preferred, such as a replication-incompetent retroviral or adenoviral vector. Pharmaceutically acceptable vectors containing the nucleic acids of this invention can be further modified for transient or stable expression of the inserted polynucleotide. As used herein, the term "pharmaceutically acceptable vector" includes, but is not limited to, a vector or delivery vehicle having the ability to selectively target and introduce the nucleic acid into dividing cells. An example of such a vector is a "replication-incompetent" vector defined by its inability to produce viral proteins, precluding spread of the vector in the infected host cell. An example of a replication-incompetent retroviral vector is LNL6 (Miller A.D. et al. (1989) BioTechniques **7**:980-990). The methodology of using replication-incompetent retroviruses for retroviral-mediated gene transfer of gene markers is well established (Correll et al. (1989) Proc. Natl. Acad. Sci. USA **86**:8912; Bordignon (1989) Proc. Natl. Acad. Sci. USA **86**:6748-6852; Culver K. (1991) Proc. Natl. Acad. Sci. USA **88**:3155; and Rill D.R. (1992) Blood **79**(10):2694-2700).

These isolated host cells containing the polynucleotides of this invention are useful for the recombinant replication of the polynucleotides and for the recombinant production of peptides. Alternatively, the cells may be used to induce an immune response in a subject in the methods described herein. When the host cells are antigen presenting cells, they can be used to expand a population of immune effector cells such as tumor infiltrating lymphocytes which in turn are useful in adoptive immunotherapies.

Also provided by this invention is an antibody capable of specifically forming a complex with the polypeptides of this invention. The term "antibody" includes polyclonal antibodies and monoclonal antibodies. The antibodies include, but are not limited to mouse, rat, and rabbit or human antibodies. The antibodies are useful to identify and purify polypeptides and APCs expressing the polypeptides.

Laboratory methods for producing polyclonal antibodies and monoclonal antibodies, as well as deducing their corresponding nucleic acid sequences, are known in the art, see Harlow and Lane (1988) *supra* and Sambrook et al. (1989) *supra.* The monoclonal antibodies of this invention can be biologically produced by introducing protein or a fragment thereof into an animal, *e.g.*, a mouse or a rabbit. The antibody producing cells in the animal are isolated and fused with myeloma cells or hetero-myeloma cells to produce hybrid cells or hybridomas. Accordingly, the hybridoma cells producing the monoclonal antibodies of this invention also are provided.

Thus, using the protein and well known methods, one of skill in the art can produce and screen the hybridoma cells and antibodies of this invention for antibodies having the ability to bind the proteins or polypeptides.

If a monoclonal antibody being tested binds with the protein or polypeptide, then the antibody being tested and the antibodies provided by the hybridomas of this invention are equivalent. It also is possible to determine without undue experimentation, whether an antibody has the same specificity as the monoclonal antibody of this invention by determining whether the antibody being tested prevents a monoclonal antibody of this invention from binding the protein or polypeptide with which the monoclonal antibody is normally reactive. If the antibody being tested competes with the monoclonal antibody of the invention as shown by a decrease in binding by the monoclonal antibody of this invention, then it is likely that the two antibodies bind to the same or a closely related epitope. Alternatively, one can pre-incubate the monoclonal antibody of this invention with a protein with which it is normally reactive, and determine if the monoclonal antibody being tested is inhibited in its ability to bind the antigen. If the monoclonal antibody being tested is inhibited then, in all likelihood, it has the same, or a closely related, epitopic specificity as the monoclonal antibody of this invention.

The term "antibody" also is intended to include antibodies of all isotypes. Particular isotypes of a monoclonal antibody can be prepared either directly by selecting from the initial fusion, or prepared secondarily, from a parental hybridoma secreting a monoclonal antibody of different isotype by using the sib selection technique to isolate class switch variants using the procedure described in Steplewski et al. (1985) Proc. Natl. Acad. Sci. USA **82**:8653 or Spira et al. (1984) J. Immunol. Meth. **74**:307.

This invention also provides biological active fragments of the polyclonal and monoclonal antibodies described above. These "antibody fragments" retain some ability to selectively bind with its antigen or immunogen. Such antibody fragments can include, but are not limited to:
(1) Fab,
(2) Fab',
(3) F(ab')₂,
(4) Fv, and
(5) SCA

A specific example of "a biologically active antibody fragment" is a CDR region of the antibody. Methods of making these fragments are known in the art, see for example, Harlow and Lane (1988) *supra*.

The antibodies of this invention also can be modified to create chimeric antibodies and humanized antibodies (Oi et al. (1986) BioTechniques **4(3)**:214). Chimeric antibodies are those in which the various domains of the antibodies' heavy and light chains are coded for by DNA from more than one species.

The isolation of other hybridomas secreting monoclonal antibodies with the specificity of the monoclonal antibodies of the invention can also be accomplished by one of ordinary skill in the art by producing anti-idiotypic antibodies (Herlyn et al. (1986) Science **232**:100). An anti-idiotypic antibody is an antibody which recognizes unique determinants present on the monoclonal antibody produced by the hybridoma of interest.

Idiotypic identity between monoclonal antibodies of two hybridomas demonstrates that the two monoclonal antibodies are the same with respect to their recognition of the same epitopic determinant. Thus, by using antibodies to the epitopic determinants on a monoclonal antibody it is possible to identify other hybridomas expressing monoclonal antibodies of the same epitopic specificity.

It is also possible to use the anti-idiotype technology to produce monoclonal antibodies which mimic an epitope. For example, an anti-idiotypic monoclonal antibody made to a first monoclonal antibody will have a binding domain in the hypervariable region which is the mirror image of the epitope bound by the first monoclonal antibody. Thus, in this instance, the anti-idiotypic monoclonal antibody could be used for immunization for production of these antibodies.

As used in this invention, the term "epitope" is meant to include any determinant having specific affinity for the monoclonal antibodies of the invention. Epitopic determinants usually consist of chemically active surface groupings of molecules such as amino acids or sugar side chains and usually have specific three dimensional structural characteristics, as well as specific charge characteristics.

The antibodies of this invention can be linked to a detectable agent or label. There are many different labels and methods of labeling known to those of ordinary skill in the art.

The coupling of antibodies to low molecular weight haptens can increase the sensitivity of the assay. The haptens can then be specifically detected by means of a second reaction. For example, it is common to use haptens such as biotin, which reacts avidin, or dinitropherryl, pyridoxal, and fluorescein, which can react with specific anti-hapten antibodies. See Harlow and Lane (1988) *supra*.

The monoclonal antibodies of the invention also can be bound to many different carriers. Thus, this invention also provides compositions containing the antibodies and another substance, active or inert. Examples of well-known carriers include glass, polystyrene, polypropylene, polyethylene, dextran, nylon, amylases, natural and modified celluloses, polyacrylamides, agaroses and magnetite. The nature of the carrier can be either soluble or insoluble for purposes of the invention. Those skilled in the art will know of other suitable carriers for binding monoclonal antibodies, or will be able to ascertain such, using routine experimentation.

Compositions containing the antibodies, fragments thereof or cell lines which produce the antibodies, are encompassed by this invention. When these compositions are to be used pharmaceutically, they are combined with a pharmaceutically acceptable carrier.

In another embodiment the present invention provides a method of inducing an immune response comprising delivering the compounds and compositions of the invention in the context of an MHC molecule. Thus, the polypeptides of this invention can be pulsed into antigen presenting cells using the methods described herein. Antigen-presenting cells, include, but are not limited to dendritic cells (DCs), monocytes/macrophages, B lymphocytes or other cell type(s) expressing the necessary MHC/co-stimulatory molecules. The methods described below focus primarily on DCs which are the most potent, preferred APCs. These host cells containing the polypeptides or proteins are further provided.

Isolated host cells which present the polypeptides of this invention in the context of MHC molecules are further useful to expand and isolate a population of educated, antigen-specific immune effector cells. The immune effector cells, *e.g*., cytotoxic T lymphocytes, are produced by culturing naïve immune effector cells with antigen-presenting cells which present the polypeptides in the context of MHC molecules on the surface of the APCs. The population can be purified using methods known in the art, *e.g.*, FACS analysis or ficoll gradient. The methods to generate and culture the immune effector cells as well as the populations produced thereby also are the inventor's contribution and invention. Pharmaceutical compositions comprising the cells and pharmaceutically acceptable carriers are useful in adoptive immunotherapy. Prior to administration *in vivo,* the immune effector cells are screened *in vitro* for their ability to lyse tumor cells expressing the human cancer antigen PAR-3, e.g., ovarian cancer.

In one embodiment, the immune effector cells and/or the APCs are genetically modified. Using standard gene transfer, genes coding for co-stimulatory molecules and/or stimulatory cytokines can be inserted prior to, concurrent to or subsequent to expansion of the immune effector cells.

This invention also provides the use of the polypeptides of the invention for the preparation of a medicament for inducing an immune response in a subject, comprising administering to the subject an effective amount of the polypeptides described above under the conditions that induce an immune response to the polypeptide. The polypeptides can be administered in formulations or as polynucleotides encoding the polypeptides. The polynucleotides can be administered in a gene delivery vehicle or by inserting into a host cell which in turn recombinantly transcribes, translates and processed the encoded polypeptide. Isolated host cells containing the polynucleotides of this invention in a pharmaceutically acceptable carrier can therefore combined with appropriate and effective amount of an adjuvant, cytokine or co-stimulatory molecule for an effective vaccine regimen. In one embodiment, the host cell is an APC such as a dendritic cell. The host cell can be further modified by inserting of a polynucleotide coding for an effective amount of either or both a cytokine and/or a co-stimulatory molecule.

The methods of this invention can be further modified by co-administering an effective amount of a cytokine or co-stimulatory molecule to the subject.

This invention also provides compositions containing any of the above-mentioned proteins, polypeptides, polynucleotides, vectors, cells, antibodies and an acceptable solid or liquid carrier. When the compositions are used pharmaceutically, they are combined with a "pharmaceutically acceptable carrier" for diagnostic and therapeutic use. These compositions also can be used for the preparation of medicaments for the diagnosis and treatment of diseases such as cancer.

The following materials and methods are intended to illustrate how to make and use the inventions described above.

### Materials and Methods

### Production of the Polypeptides of the Invention

Most preferably, isolated peptides of the present invention can be synthesized using an appropriate solid state synthetic procedure. Steward and Young, SOLID PHASE PEPTIDE SYNTHESIS, Freemantle, San Francisco, Calif. (1968). A preferred method is the Merrifield process. See, Merrifield (1967) Recent Progress in Hormone Res. 23:451. The antigenic activity of these peptides may conveniently be tested using, for example, the assays as described herein.

Once an isolated peptide of the invention is obtained, it may be purified by standard methods including chromatography *(e.g.,* ion exchange, affinity, and sizing column chromatography), centrifugation, differential solubility, or by any other standard technique for protein purification. For immuno-affinity chromatography, an epitope may be isolated by binding it to an affinity column comprising antibodies that were raised against that peptide, or a related peptide of the invention, and were affixed to a stationary support.

Alternatively, affinity tags such as hexa-His (Invitrogen), Maltose binding domain (New England Biolabs), influenza coat sequence (Kolodziej et al. (1991) Meth. Enzymol. **194**:508-509), and glutathione-S-transferase can be attached to the peptides of the invention to allow easy purification by passage over an appropriate affinity column. Isolated peptides can also be physically characterized using such techniques as proteolysis, nuclear magnetic resonance, and x-ray crystallography.

Also included within the scope of the invention are antigenic peptides that are differentially modified during or after translation, *e.g*., by phosphorylation, glycosylation, cross-linking, acylation, proteolytic cleavage, linkage to an antibody molecule, membrane molecule or other ligand, (Ferguson et al. (1988) Ann. Rev. Biochem. **57**:285-320).

### Isolation, Culturing and Expansion of APCs, Including Dendritic Cells

The following is a brief description of two fundamental approaches for the isolation of APC. These approaches involve (1) isolating bone marrow precursor cells (CD34⁺) from blood and stimulating them to differentiate into APC; or (2) collecting the precommitted APCs from peripheral blood. In the first approach, the patient must be treated with cytokines such as GM-CSF to boost the number of circulating CD34⁺ stem cells in the peripheral blood.

The second approach for isolating APCs is to collect the relatively large numbers of precommitted APCs already circulating in the blood. Previous techniques for isolating committed APCs from human peripheral blood have involved combinations of physical procedures such as metrizamide gradients and adherence/non-adherence steps (Freudenthal P.S. et al. (1990) Proc. Natl. Acad. Sci. USA **87**:7698-7702); Percoll gradient separations (Mehta-Damani et al. (1994) J. Immunol. **153**:996-1003); and fluorescence activated cell sorting techniques (Thomas R. et al. (1993) J. Immunol. **151**:6840-6852).

One technique for separating large numbers of cells from one another is known as countercurrent centrifugal elutriation (CCE). In this technique, cells are subject to simultaneous centrifugation and a washout stream of buffer that is constantly increasing in flow rate. The constantly increasing countercurrent flow of buffer leads to fractional cell separations that are largely based on cell size.

In one aspect of the invention, the APC are precommitted or mature dendritic cells which can be isolated from the white blood cell fraction of a mammal, such as a murine, simian or a human (See, *e.g.,* WO 96/23060). The white blood cell fraction can be from the peripheral blood of the mammal. This method includes the following steps: (a) providing a white blood cell fraction obtained from a mammalian source by methods known in the art such as leukophoresis; (b) separating the white blood cell fraction of step (a) into four or more subfractions by countercurrent centrifugal elutriation; (c) stimulating conversion of monocytes in one or more fractions from step (b) to dendritic cells by contacting the cells with calcium ionophore, GM-CSF and IL-13 or GM-CSF and IL-4, (d) identifying the dendritic cell-enriched fraction from step (c); and (e) collecting the enriched fraction of step (d), preferably at about 4°C. One way to identify the dendritic cell-enriched fraction is by fluorescence-activated cell sorting. The white blood cell fraction can be treated with calcium ionophore in the presence of other cytokines, such as recombinant (rh) rhIL-12, rhGM-CSF, or rhIL-4. The cells of the white blood cell fraction can be washed in buffer and suspended in Ca⁺⁺/Mg⁺⁺ free media prior to the separating step. The white blood cell fraction can be obtained by leukapheresis. The dendritic cells can be identified by the presence of at least one of the following markers: HLA-DR, HLA-DQ, or B7. 2, and the simultaneous absence of the following markers: CD3, CD 14, CD16, 56, 57, and CD 19, 20. Monoclonal antibodies specific to these cell surface markers are commercially available.

More specifically, the method requires collecting an enriched collection of white cells and platelets from leukapheresis that is then further fractionated by countercurrent centrifugal elutriation (CCE) (Abrahamsen T.G. et al. (1991) J. Clin. Apheresis. **6**:48-53). Cell samples are placed in a special elutriation rotor. The rotor is then spun at a constant speed of, for example, 3000 rpm. Once the rotor has reached the desired speed, pressurized air is used to control the flow rate of cells. Cells in the elutriator are subjected to simultaneous centrifugation and a washout stream of buffer that is constantly increasing in flow rate. This results in fractional cell separations based largely but not exclusively on differences in cell size.

Quality control of APC and more specifically DC collection and confirmation of their successful activation in culture is dependent upon a simultaneous multi-color FACS analysis technique which monitors both monocytes and the dendritic cell subpopulation as well as possible contaminant T lymphocytes. It is based upon the fact that DCs do not express the following markers: CD3 (T cell); CD14 (monocyte); CD16, 56, 57 (NK/LAK cells); CD19, 20 (B cells). At the same time, DCs do express large quantities of HLA-DR, significant HLA-DQ and B7.2 (but little or no B7.1) at the time they are circulating in the blood (in addition they express Leu M7 and M9, myeloid markers which are also expressed by monocytes and neutrophils).

When combined with a third color reagent for analysis of dead cells, propridium iodide (PI), it is possible to make positive identification of all cell subpopulations (see Table 1):

**TABLE 1**

| FACS analysis of fresh peripheral cell subpopulations | | | |
|---|---|---|---|
| | Color #1 | Color #2 | Color #3 |
| | Cocktail 3/14/16/19/20/56/57 | HLA-DR | PI |
| | | | |
| Live Dendritic cells | Negative | Positive | Negative |
| Live Monocytes | Positive | Positive | Negative |
| Live Neutrophils | Negative | Negative | Negative |
| Dead Cells | Variable | Variable | Positive |

Additional markers can be substituted for additional analysis:
Color #1: CD3 alone, CD14 alone, etc.; Leu M7 or Leu M9; anti-Class I, etc.
Color #2: HLA-DQ, B7.1, B7.2, CD25 (IL2r), ICAM, LFA-3, etc.

The goal of FACS analysis at the time of collection is to confirm that the DCs are enriched in the expected fractions, to monitor neutrophil contamination, and to make sure that appropriate markers are expressed. This rapid bulk collection of enriched DCs from human peripheral blood, suitable for clinical applications, is absolutely dependent on the analytic FACS technique described above for quality control. If need be, mature DCs can be immediately separated from monocytes at this point by fluorescent sorting for "cocktail negative" cells. It may not be necessary to routinely separate DCs from monocytes because, as will be detailed below, the monocytes themselves are still capable of differentiating into DCs or functional DC-like cells in culture.

Once collected, the DC rich/monocyte APC fractions (usually 150 through 190) can be pooled and cryopreserved for future use, or immediately placed in short term culture.

Alternatively, others have reported a method for upregulating (activating) dendritic cells and converting monocytes to an activated dendritic cell phenotype. This method involves the addition of calcium ionophore to the culture media convert monocytes into activated dendritic cells. Adding the calcium ionophore A23187, for example, at the beginning of a 24-48 hour culture period resulted in uniform activation and dendritic cell phenotypic conversion of the pooled "monocyte plus DC" fractions: characteristically, the activated population becomes uniformly CD14 (Leu M3) negative, and upregulates HLA-DR, HLA-DQ, ICAM-1, B7.1, and B7.2. Furthermore, this activated bulk population functions as well on a small numbers basis as a further purified.

Specific combination(s) of cytokines have been used successfully to amplify (or partially substitute) for the activation/conversion achieved with calcium ionophore: these cytokines include but are not limited to purified or recombinant ("rh") rhGM-CSF, rhIL-2, and rhIL-4. Each cytokine when given alone is inadequate for optimal upregulation.

### Presentation of Antigen to the APC

For purposes of immunization, the antigenic peptides (Nos. 3, 5, 7, 9 and 11) can be delivered to antigen-presenting cells as protein/peptide or in the form of cDNA encoding the protein/peptide. Antigen-presenting cells (APCs) can consist of dendritic cells (DCs), monocytes/macrophages, B lymphocytes or other cell type(s) expressing the necessary MHC/co-stimulatory molecules. The methods described below focus primarily on DCs which are the most potent, preferred APCs.

Pulsing is accomplished *in vitro*/*ex vivo* by exposing APCs to the antigenic protein or peptide(s) of this invention. The protein or peptide(s) are added to APCs at a concentration of 1-10 µm for approximately 3 hours. Pulsed APCs can subsequently be administered to the host via an intravenous, subcutaneous, intranasal, intramuscular or intraperitoneal route of delivery.

Protein/peptide antigen can also be delivered *in vivo* with adjuvant via the intravenous, subcutaneous, intranasal, intramuscular or intraperitoneal route of delivery.

Paglia et al. (1996) J. Exp. Med. **183**:317-322 has shown that APC incubated with whole protein *in vitro* were recognized by MHC class I-restricted CTLs, and that immunization of animals with these APCs led to the development of antigen-specific CTLs *in vivo*. In addition, several different techniques have been described which lead to the expression of antigen in the cytosol of APCs, such as DCs. These include (1) the introduction into the APCs of RNA isolated from tumor cells, (2) infection of APCs with recombinant vectors to induce endogenous expression of antigen, and (3) introduction of tumor antigen into the DC cytosol using liposomes. (See Boczkowski D. et al. (1996) J. Exp. Med. **184**:465-472; Rouse et al. (1994) J. Virol. **68**:5685-5689; and Nair et al. (1992) J. Exp. Med. **175**:609-612).

### Foster Antigen Presenting Cells

Foster antigen presenting cells are particularly useful as target cells. Foster APCs are derived from the human cell line 174xCEM.T2, referred to as T2, which contains a mutation in its antigen processing pathway that restricts the association of endogenous peptides with cell surface MHC class I molecules (Zweerink et al. (1993) J. Immunol. **150**:1763-1771). This is due to a large homozygous deletion in the MHC class II region encompassing the genes TAP1, TAP2, LMP1, and LMP2, which are required for antigen presentation to MHC class 1-restricted CD8⁺ CTLs. In effect, only "empty" MHC class I molecules are presented on the surface of these cells. Exogenous peptide added to the culture medium binds to these MHC molecules provided that the peptide contains the allele-specific binding motif. These T2 cells are referred to herein as "foster" APCs. They can be used in conjunction with this invention to present antigen(s).

Transduction of T2 cells with specific recombinant MHC alleles allows for redirection of the MHC restriction profile. Libraries tailored to the recombinant allele will be preferentially presented by them because the anchor residues will prevent efficient binding to the endogenous allele.

High level expression of MHC molecules makes the APC more visible to the CTLs. Expressing the MHC allele of interest in T2 cells using a powerful transcriptional promoter (*e.g*., the CMV promoter) results in a more reactive APC (most likely due to a higher concentration of reactive MHC-peptide complexes on the cell surface).

### Immunogenicity Assays.

The immunogenicity of invention ligands can be determined by well known methodologies including, but not limited to those exemplified below. In one embodiment, such methodology may be employed to compare an altered ligand of the invention with the corresponding native ligand. For example, an altered ligand may be considered "more active" if it compares favorably with the activity of the native ligand in any one of the following assays. For some purposes, one skilled in the art will select an immunogenic ligand which displays more activity than another immunogenic ligand, *i.e.,* for treatment and/or diagnostic purposes. However, for some applications, the use of an immunogenic ligand which is comparable with the native ligand will be suitable. In other situations, it may be desirable to utilize an immunogenic ligand which is less active. It has been suggested that such levels of activity positively correlate with the level of immunogenicity.
1. ^{**51**}**Cr-release lysis assay.** Lysis of peptide-pulsed ⁵¹Cr-labeled targets by antigen-specific T cells can be compared for target cells pulsed with either the native or altered ligands. Functionally enhanced ligands will show greater lysis of targets as a function of time. The kinetics of lysis as well as overall target lysis at a fixed timepoint (e.g., 4 hours) may be used to evaluate ligand performance. (Ware C.F. et al. (1983) J. Immunol. **131**:1312).
2. **Cytokine-release assay.** Analysis of the types and quantities of cytokines secreted by T cells upon contacting ligand-pulsed targets can be a measure of functional activity. Cytokines can be measured by ELISA or ELISPOT assays to determine the rate and total amount of cytokine production. (Fujihashi K. et al. (1993) J. Immunol. Meth. 160:181; Tanguay S. and Killion J.J. (1994) Lymphokine Cytokine Res. **13**:259).
3. ***In vitro* T cell education.** The ligands of the invention can be compared to the corresponding native ligand for the ability to elicit ligand-reactive T cell populations from normal donor or patient-derived PBMC. In this system, elicited T cells can be tested for lytic activity, cytokine-release, polyclonality, and cross-reactivity to the native ligand. (Parkhurst M.R. et al. (1996) J. Immunol. **157**:2539).
4. **Transgenic animal models.** Immunogenicity can be assessed *in vivo* by vaccinating HLA transgenic mice with either the ligands of the invention or the native ligand and determining the nature and magnitude of the induced immune response. Alternatively, the hu-PBL-SCID mouse model allows reconstitution of a human immune system in a mouse by adoptive transfer of human PBL. These animals may be vaccinated with the ligands and analyzed for immune response as previously mentioned. (Shirai M. et al. (1995) J. Immunol. **154**:2733; Mosier D.E. et al. (1993) Proc. Natl. Acad. Sci USA **90**:2443).
5. **Proliferation.** T cells will proliferate in response to reactive ligands. Proliferation can be monitored quantitatively by measuring, for example, ³H-thymidine uptake. (Caruso A. et al. (1997) Cytometry **27**:71).
6. **Tetramer staining.** MHC tetramers can be loaded with individual ligands and tested for their relative abilities to bind to appropriate effector T cell populations. (Altman J.D. et al. (1996) Science **274(5284)**:94-96.
7. **MHC Stabilization.** Exposure of certain cell lines such as T2 cells to HLA-binding ligands results in the stabilization of MHC complexes on the cell surface. Quantitation of MHC complexes on the cell surface has been correlated with the affinity of the ligand for the HLA allele that is stabilized. Thus, this technique can determine the relative HLA affinity of ligand epitopes. (Stuber G. et al. (1995) Int. Immunol. **7**:653).
8. **MHC competition.** The ability of a ligand to interfere with the functional activity of a reference ligand and its cognate T cell effectors is a measure of how well a ligand can compete for MHC binding. Measuring the relative levels of inhibition is an indicator of MHC affinity. (Feltkamp M.C. et al. (1995) Immunol. Lett. **47**:1).
9. **Primate models.** A recently described non-human primate (chimpanzee) model system can be utilized to monitor *in vivo* immunogenicities of HLA-restricted ligands. It has been demonstrated that chimpanzees share overlapping MHC-ligand specificities with human MHC molecules thus allowing one to test HLA-restricted ligands for relative *in vivo* immunogenicity. (Bertoni R. et al. (1998) J. Immunol. **161**:4447).
10. **Monitoring TCR Signal Transduction Events.** Several intracellular signal transduction events (e.g., phosphorylation) are associated with successful TCR engagement by MHC-ligand complexes. The qualitative and quantitative analysis of these events have been correlated with the relative abilities of ligands to activate effector cells through TCR engagement. (Salazar E. et al. (2000) Int. J. Cancer 85:829; Isakov N. et al. (1995) J. Exp. Med. **181**:375).

### Expansion of Immune Effector Cells

The present invention makes use of these APCs to stimulate production of an enriched population of antigen-specific immune effector cells. The antigen-specific immune effector cells are expanded at the expense of the APCs, which die in the culture. The process by which naive immune effector cells become educated by other cells is described essentially in Coulie (1997) Molec. Med. Today **3**:261-268.

The APCs prepared as described above are mixed with naïve immune effector cells. Preferably, the cells may be cultured in the presence of a cytokine, for example IL2. Because dendritic cells secrete potent immunostimulatory cytokines, such as IL12, it may not be necessary to add supplemental cytokines during the first and successive rounds of expansion. In any event, the culture conditions are such that the antigen-specific immune effector cells expand (*i*.*e*., proliferate) at a much higher rate than the APCs. Multiple infusions of APCs and optional cytokines can be performed to further expand the population of antigen-specific cells.

In one embodiment, the immune effector cells are T cells. In a separate embodiment, the immune effector cells can be genetically modified by transduction with a transgene coding for example, IL-2, IL-11 or IL-13. Methods for introducing transgenes *in vitro, ex vivo* and *in vivo* are well known in the art. See Sambrook et al. (1989) *supra.*

### Vectors Useful in Genetic Modifications

In general, genetic modifications of cells employed in the present invention are accomplished by introducing a vector containing a polypeptide or transgene encoding a heterologous or an altered antigen. A variety of different gene transfer vectors, including viral as well as non-viral systems can be used. Viral vectors useful in the genetic modifications of this invention include, but are not limited to adenovirus, adeno-associated virus vectors, retroviral vectors and adeno-retroviral chimeric vectors. APC and immune effector cells can be modified using the methods described below or by any other appropriate method known in the art.

### Construction of Recombinant Adenoviral Vectors or Adeno-Associated Virus Vectors

Adenovirus and adeno-associated virus vectors useful in the genetic modifications of this invention may be produced according to methods already taught in the art. See, *e.g.,* Karlsson et al. (1986) EMBO J. **5**:2377; Carter (1992) Curr. Op. Biotechnol. 3:533-539; Muzcyzka (1992) Current Top. Microbiol. Immunol. **158**:97-129; GENE TARGETING: A PRACTICAL APPROACH (1992) ed. A. L. Joyner, Oxford University Press, NY). Several different approaches are feasible. Preferred is the helper-independent replication deficient human adenovirus system.

The recombinant adenoviral vectors based on the human adenovirus 5 (McGrory, W.T., et al. (1998) Virology **163**:614-617) are missing essential early genes from the adenoviral genome (usually E1A/E1B), and are therefore unable to replicate unless grown in permissive cell lines that provide the missing gene products *in trans.* In place of the missing adenoviral genomic sequences, a transgene of interest can be cloned and expressed in cells infected with the replication deficient adenovirus. Although adenovirus-based gene transfer does not result in integration of the transgene into the host genome (less than 0.1% adenovirus-mediated transfections result in transgene incorporation into host DNA), and therefore is not stable, adenoviral vectors can be propagated in high titer and transfect non-replicating cells. Human 293 cells, which are human embryonic kidney cells transformed with adenovirus E1A/E1B genes, typify useful permissive cell lines. However, other cell lines which allow replication-deficient adenoviral vectors to propagate therein can be used, including HeLa cells.

Additional references describing adenovirus vectors and other viral vectors which could be used in the methods of the present invention include the following: Horwitz M.S. ADENOVIRIDAE AND THEIR REPLICATION, in Fields B. et al. (eds.) VIROLOGY, Vol. 2, Raven Press New York, pp. 1679-1721 (1990); Graham F. et al. pp. 109-128 in METHODS IN MOLECULAR BIOLOGY, Vol. 7: GENE TRANSFER AND EXPRESSION PROTOCOLS, Murray E. (ed.) Humana Press, Clifton, N.J. (1991); Miller N. et al. (1995) FASEB J. **9**:190-199; Schreier H. (1994) Pharmaceutica Acta Helvetiae **68**:145-159; Schneider and French (1993) Circulation **88**:1937-1942; Curiel D.T. et al.(1992) Hum. Gene Ther. **3**:147-154; Graham F.L. et al. WO 95/00655 (5 January 1995); Falck-Pedersen E.S. WO 95/16772 (22 June 1995); Denefle P. et al. WO 95/23867 (8 September 1995); Haddada H. et al. WO 94/26914 (24 November 1994); Perricaudet M. et al. WO 95/02697 (26 January 1995); Zhang W. et al. WO 95/25071 (12 October 1995). A variety of adenovirus plasmids are also available from commercial sources, including, *e.g.,* Microbix Biosystems of Toronto, Ontario (see, *e.g.,* Microbix Product Information Sheet: Plasmids for Adenovirus Vector Construction, 1996). See also, the papers by Vile et al. (1997) Nature Biotechnology **15**:840-841; and Feng et al. (1997) Nature Biotechnology **15**:866-870, describing the construction and use of adeno-retroviral chimeric vectors that can be employed for genetic modifications.

Additional references describing AAV vectors that could be used in the methods of the present invention include the following: Carter B. HANDBOOK OF PARVOVIRUSES, Vol. I, pp. 169-228,1990; Berns, VIROLOGY, pp. 1743-1764 (Raven Press 1990); Carter B. (1992) Curr. Opin. Biotechnol. **3**:533-539; Muzyczka N. (1992) Current Topics in Micro. and Immunol, **158**:92-129; Flotte T.R. et al. (1992) Am. J. Respir. Cell Mol. Biol. **7**:349-356; Chatterjee et al. (1995) Ann. NY Acad. Sci. **770**:79-90; Flotte T.R. et al. WO 95/13365 (18 May 1995); Trempe J.P. et al., WO 95/13392 (18 May 1995); Kotin R.(1994) Hum. Gene Ther. **5**:793-801; Flotte T.R. et al. (1995) Gene Therapy **2**:357-362; Allen J.M. WO 96/17947 (13 June 1996); and Du et al. (1996) Gene Therapy **3**:254-261.

APCs can be transduced with viral vectors encoding a relevant polypeptides. The most common viral vectors include recombinant poxviruses such as vaccinia and fowlpox virus (Bronte et al. (1997) Proc. Natl. Acad. Sci. USA **94**:3183-3188; Kim et al. (1997) J. Immunother. **20**:276-286) and, preferentially, adenovirus (Arthur et al. (1997) J. Immunol. **159**:1393-1403; Wan et al. (1997) Human Gene Therapy **8**:1355-1363; Huang et al. (1995) J. Virol. **69**:2257-2263). Retrovirus also may be used for transduction of human APCs (Marin et al. (1996) J. Virol. **70**:2957-2962).

*In vitro*/*ex vivo,* exposure of human DCs to adenovirus (Ad) vector at a multiplicity of infection (MOI) of 500 for 16-24 h in a minimal volume of serum-free medium reliably gives rise to transgene expression in 90-100% of DCs. The efficiency of transduction of DCs or other APCs can be assessed by immunofluorescence using fluorescent antibodies specific for the tumor antigen being expressed (Kim et al. (1997) J. Immunother. **20**:276-286). Alternatively, the antibodies can be conjugated to an enzyme (*e*.*g*., HRP) giving rise to a colored product upon reaction with the substrate. The actual amount of antigenic polypeptides being expressed by the APCs can be evaluated by ELISA.

Transduced APCs can subsequently be administered to the host via an intravenous, subcutaneous, intranasal, intramuscular or intraperitoneal route of delivery.

*In vivo* transduction of DCs, or other APCs, can be accomplished by administration of Ad (or other viral vectors) via different routes including intravenous, intramuscular, intranasal, intraperitoneal or cutaneous delivery. The preferred method is cutaneous delivery of Ad vector at multiple sites using a total dose of approximately 1x10¹⁰-1x 10¹² i.u. Levels of *in vivo* transduction can be roughly assessed by co-staining with antibodies directed against APC marker(s) and the TAA being expressed. The staining procedure can be carried out on biopsy samples from the site of administration or on cells from draining lymph nodes or other organs where APCs (in particular DCs) may have migrated (Condon et al. (1996) Nature Med. **2**:1122-1128 and Wan et al. (1997) Hum. Gene Ther. **8**:1355-1363). The amount of antigen being expressed at the site of injection or in other organs where transduced APCs may have migrated can be evaluated by ELISA on tissue homogenates.

Although viral gene delivery is more efficient, DCs can also be transduced *in vitro*/*ex vivo* by non-viral gene delivery methods such as electroporation, calcium phosphate precipitation or cationic lipid/plasmid DNA complexes (Arthur et al. (1997) Cancer Gene Ther. **4**:17-25). Transduced APCs can subsequently be administered to the host via an intravenous, subcutaneous, intranasal, intramuscular or intraperitoneal route of delivery.

*In vivo* transduction of DCs, or other APCs, can potentially be accomplished by administration of cationic lipid/plasmid DNA complexes delivered via the intravenous, intramuscular, intranasal, intraperitoneal or cutaneous route of administration. Gene gun delivery or injection of naked plasmid DNA into the skin also leads to transduction of DCs (Condon et al. (1996) Nature Med. **2**:1122-1128; Raz et al (1994) Proc. Natl. Acad. Sci. USA **91**:9519-9523). Intramuscular delivery of plasmid DNA may also be used for immunization (Rosato et al. (1997) Hum. Gene Ther. **8**:1451-1458.)

The transduction efficiency and levels of transgene expression can be assessed as described above for viral vectors.

### Adoptive Immunotherapy and Vaccines

The expanded populations of antigen-specific immune effector cells of the present invention also find use in adoptive immunotherapy regimes and as vaccines.

Adoptive immunotherapy methods involve, in one aspect, administering to a subject a substantially pure population of educated, antigen-specific immune effector cells made by culturing naïve immune effector cells with APCs as described above. Preferably, the APCs are dendritic cells.

In one embodiment, the adoptive immunotherapy methods described herein are autologous. In this case, the APCs are made using parental cells isolated from a single subject. The expanded population also employs T cells isolated from that subject. Finally, the expanded population of antigen-specific cells is administered to the same patient.

In a further embodiment, APCs or immune effector cells are administered with an effective amount of a stimulatory cytokine, such as IL-2 or a co-stimulatory molecule.

The agents identified herein as effective for their intended purpose can be administered to subjects having tumors expressing human cancer tumor antigen PAR-3 as well as or in addition to individuals susceptible to or at risk of developing such tumors. When the agent is administered to a subject such as a mouse, a rat or a human patient, the agent can be added to a pharmaceutically acceptable carrier and systemically or topically administered to the subject. To determine patients that can be beneficially treated, a tumor regression can be assayed. Therapeutic amounts can be empirically determined and will vary with the pathology being treated, the subject being treated and the efficacy and toxicity of the therapy.

Administration *in vivo* can be effected in one dose, continuously or intermittently throughout the course of treatment. Methods of determining the most effective means and dosage of administration are well known to those of skill in the art and will vary with the composition used for therapy, the purpose of the therapy, the target cell being treated, and the subject being treated. Single or multiple administrations can be carried out with the dose level and pattern being selected by the treating physician. Suitable dosage formulations and methods of administering the agents can be found below.

The agents and compositions of the present invention can be used in the manufacture of medicaments for the treatment of humans and other animals by administration in accordance with conventional procedures, such as an active ingredient in pharmaceutical compositions.

More particularly, an agent of the present invention also referred to herein as the active ingredient, may be administered for therapy by any suitable route including nasal, topical (including transdermal, aerosol, buccal and sublingual), parental (including subcutaneous, intramuscular, intravenous and intradermal) and pulmonary. It will also be appreciated that the preferred route will vary with the condition and age of the recipient, and the disease being treated.

## Claims

1. A compound having the structure selected from the group of

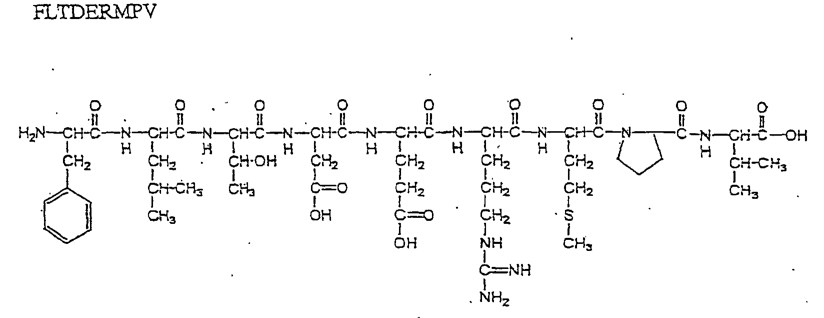

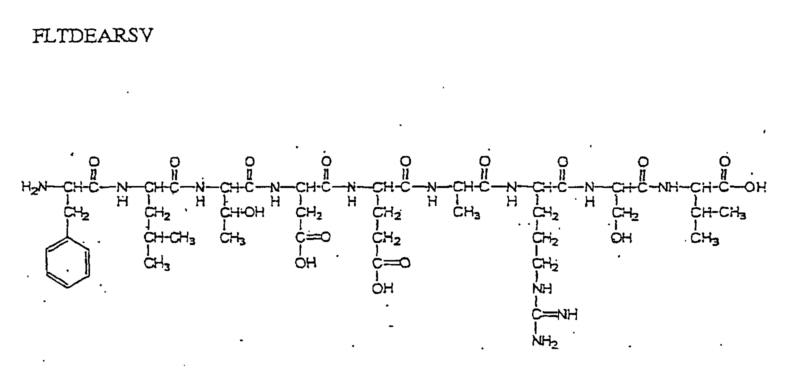

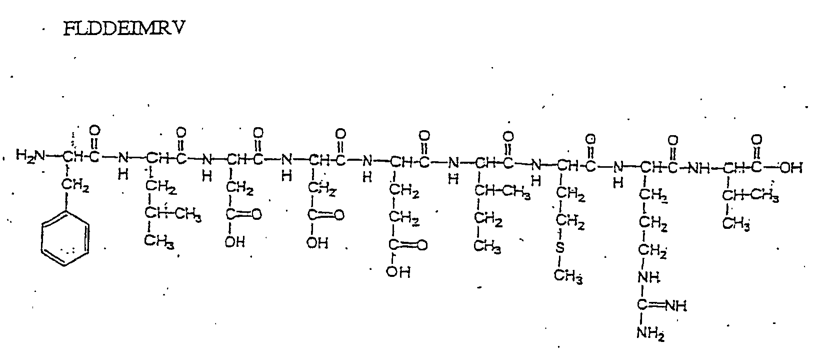

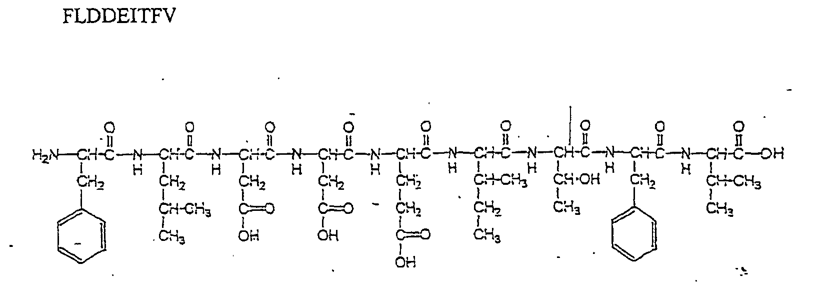

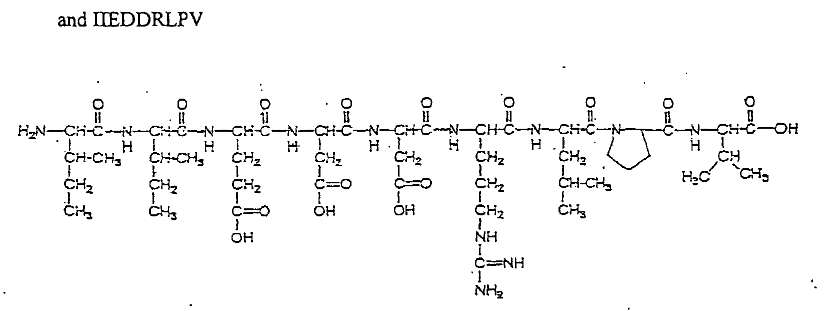

2. A peptide comprising the amino acid sequence of SEQ ID NO:2, wherein amino acids 700, 701, 702, 704 and 706 are F, L, T, E, and M, respectively.

3. A peptide comprising the amino acid sequence of SEQ ID NO:2, wherein amino acids 700, 701, 702, 704, 705, 706, and 707 are F, L, D, E, I, T and F, respectively, or F, L, T, E, A, R, and S, respectively, or F, L, D, E, I, M and R, respectively.

4. A polynucleotide that encodes an amino acid sequence comprising the amino acids of SEQ ID NO:3, SEQ ID NO:5, SEQ ID NO:7, or SEQ ID NO:9.

5. An antibody that specifically recognizes and binds a compound of claim 1.

6. Use of an effective amount of a compound of claim 1 for the preparation of a medicament for inducing an immune response in a subject.

7. Use according to claim 7, wherein the compound is delivered in the context of an MHC molecule, or as a polynucleotide that encodes the compound.

8. Use according to claim 7 or 8, wherein the MHC molecule presents the compound on the surface of an antigen presenting cell.

9. Use of an effective amount of an antibody of claim 6 for the preparation of a medicament for immunotherapy in a subject.

10. An immune effector cell that has been raised *in vitro* or *in vivo* in the presence and at the expense of an antigen presenting cell that presents a compound of claim 1 in the context of an MHC molecule.

11. Use of an effective amount of the immune effector cell of claim 11 for the preparation of a medicament for adoptive immunotherapy in a subject.

12. A composition comprising at least two immunogenic ligands, wherein said immunogenic ligands are individually **characterized by** an ability to elicit an immune response against the same native ligand, and wherein said immunogenic ligand is selected from the group consisting of FLTDERMPV (SEQ ID NO:3), FLTDEARSV (SEQ ID NO:5), FLDDEIMRV (SEQ ID NO:7), FLDDEITFV (SEQ ID NO:9) and IIEDDRLPV (SEQ ID NO:11).

13. The composition of claim 13, further comprising a biologically active immunoglobulin variable domain bound to said immunogenic ligands.

14. The composition of claim 13, further comprising an MHC molecule bound to said immunogenic ligands.

15. The composition of claim 13, wherein said immunogenic ligands are linked covalently.

16. The composition of claim 13; further comprising an agent covalently linked to said immunogenic ligands, wherein said agent is capable of targeting said immunogenic ligands to an antigen presenting cell, preferably a dendritic cell.

17. The composition of claim 13, further comprising an MHC class II binding helper peptide.

18. The composition of any of claims 13 to 18, further comprising a carrier, preferably a pharmaceutically acceptable carrier.

19. A host cell comprising at least two immunogenic ligands, wherein said immunogenic ligands are individually **characterized by** an ability to elicit an immune response against the same native ligand, and wherein said immunogenic ligand is selected from the group consisting of FLTDERMPV (SEQ ID NO:3), FLTDEARSV (SEQ ID NO:5), FLDDEIMRV (SEQ ID NO:7), FLDDEITFV (SEQ ID NO:9) and IIEDDRLPV (SEQ ID NO:11).

20. The host cell of claim 20, wherein the host cell is an antigen presenting cell, preferably a dendritic cell, and the immunogenic ligands are presented on the surface of the cell.

21. A composition comprising the host cell of claim 20 or 21 and a carrier, preferably a pharmaceutically acceptable carrier.

22. Use of an effective amount of two or more immunogenic ligands, wherein each of said immunogenic ligands is **characterized by** an ability to elicit an immune response against the same native ligand, and wherein said immunogenic ligand is selected from the group consisting of FLTDERMPV (SEQ ID NO:3), FLTDEARSV (SEQ ID NO:5), FLDDEIMRV (SEQ ID NO:7), FLDDEITFV (SEQ ID NO:9) and IIEDDRLPV (SEQ ID NO:11) for the preparation of a medicament for inducing an immune response in a subject.

## Patentansprüche

1. Verbindung mit der Struktur ausgewählt aus der Gruppe von

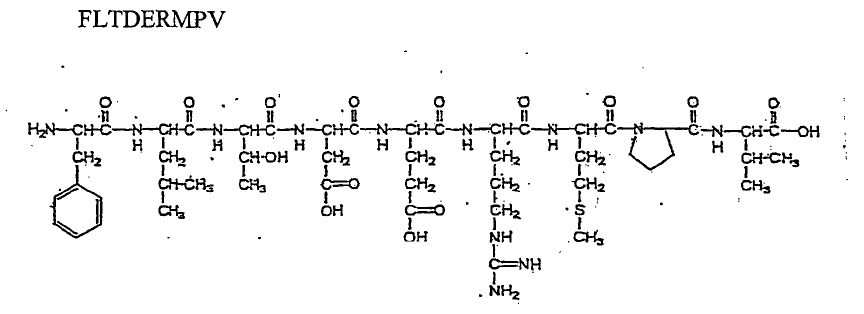

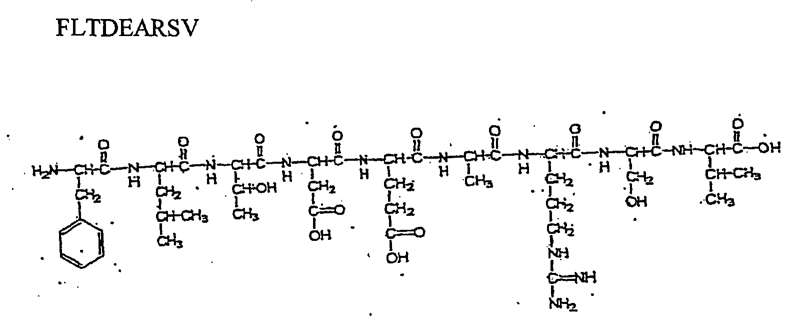

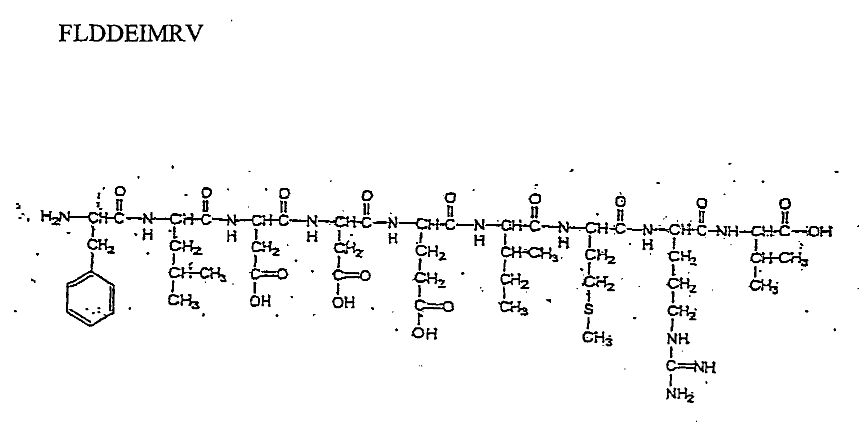

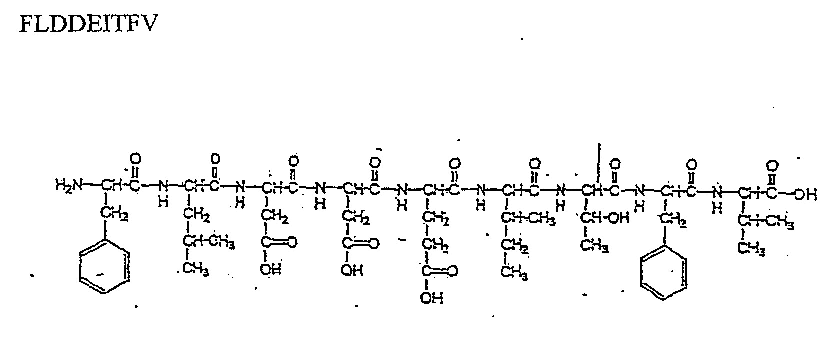

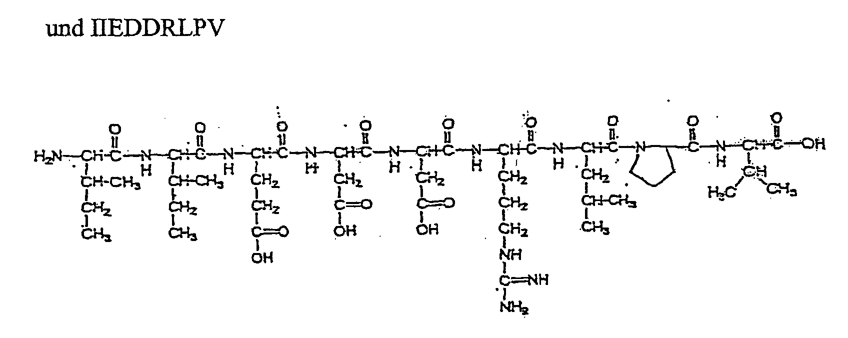

2. Peptid, umfassend die Aminosäuresequenz von SEQ ID NO:2, wobei die Aminosäuren 700, 701, 702, 704 und 706 F, L, T, E bzw. M sind.

3. Peptid, umfassend die Aminosäuresequenz von SEQ ID NO:2, wobei die Aminosäuren 700, 701, 702, 704, 705, 706 und 707 F, L, D, E, I, T bzw. F oder F, L, T, E, A, R bzw. S oder F, L, D, E, I, M bzw. R sind.

4. Polynukleotid, welches eine Aminosäuresequenz kodiert, die die Aminosäuren von SEQ ID NO:3, SEQ ID NO:5, SEQ ID NO:7 oder SEQ ID NO: 9 umfasst.

5. Antikörper, welcher eine Verbindung gemäß Anspruch 1 spezifisch erkennt und bindet.

6. Verwendung einer wirksamen Menge einer Verbindung gemäß Anspruch 1 zur Herstellung eines Medikaments zur Induktion einer Immunantwort in einem Subjekt.

7. Verwendung gemäß Anspruch 6, wobei die Verbindung im Kontext von einem MHC-Molekül oder als ein Polynukleotid, welches die Verbindung kodiert, gegeben wird.

8. Verwendung gemäß Anspruch 6 oder 7, wobei das MHC-Molekül die Verbindung auf der Oberfläche einer antigenpräsentierenden Zelle präsentiert.

9. Verwendung einer wirksamen Menge eines Antikörpers gemäß Anspruch 5 zur Herstellung eines Medikaments zur Immuntherapie in einem Subjekt.

10. Immuneffektorzelle, *welche in vitro* oder *in vivo* in Anwesenheit und auf Kosten einer antigenpräsentierenden Zelle erzeugt wurde, welche eine Verbindung gemäß Anspruch 1 im Kontext von einem MHC-Molekül präsentiert.

11. Verwendung einer wirksamen Menge der Immuneffektorzelle gemäß Anspruch 10 zur Herstellung eines Medikaments zur adoptiven Immuntherapie in einem Subjekt.

12. Zusammensetzung, mindestens zwei immunogene Liganden umfassend, wobei die immunogenen Liganden durch eine Fähigkeit, eine Immunantwort gegen den gleichen nativen Liganden auszulösen, individuell charakterisiert sind, und wobei die immunogenen Liganden aus der Gruppe ausgewählt sind, bestehend aus
FLTDERMPV (SEQ ID NO:3), FLTDEARSV (SEQ ID NO:5),
FLDDEIMRV (SEQ ID NO:7), FLDDEITFV (SEQ ID NO:9) und
IIEDDRLPV (SEQ ID NO:11).

13. Zusammensetzung gemäß Anspruch 12, weiterhin umfassend eine biologisch aktive, variable Immunoglobin-Domäne, die an die immunogenen Liganden gebunden ist.

14. Zusammensetzung gemäß Anspruch 12, weiterhin umfassend ein MHC-Molekül, das an die immunogenen Liganden gebunden ist.

15. Zusammensetzung gemäß Anspruch 12, wobei die immunogenen Liganden kovalent verbunden sind.

16. Zusammensetzung gemäß Anspruch 12, weiterhin umfassend einen an die immunogenen Liganden kovalent gebundenen Wirkstoff, wobei der Wirkstoff geeignet ist, die immunogenen Liganden an eine antigenpräsentierende Zelle, vorzugsweise eine dentritische Zelle, zu richten.

17. Zusammensetzung gemäß Anspruch 12, weiterhin umfassend ein MHC-Klasse2-bindendes Helferpeptid.

18. Zusammensetzung gemäß einem der Ansprüche 12 bis 17, weiterhin umfassend einen Träger, vorzugsweise einen pharmazeutisch akzeptablen Träger.

19. Gastzelle, mindestens zwei immunogene Liganden umfassend, wobei die immunogenen Liganden durch eine Fähigkeit, eine Immunantwort gegen den gleichen nativen Liganden auszulösen, individuell charakterisiert sind, und wobei jeder der immunogenen Liganden aus der Gruppe ausgewählt ist, bestehend aus
FLTDERMPV (SEQ ID NO:3), FLTDEARSV (SEQ ID NO:5),
FLDDEIMRV (SEQ ID NO:7), FLDDEITFV (SEQ ID NO:9) und
IIEDDRLPV (SEQ ID NO:11).

20. Gastzelle gemäß Anspruch 19, wobei die Gastzelle eine antigenpräsentierende Zelle, vorzugsweise eine dentritische Zelle, ist und die immunogenen Liganden auf der Oberfläche der Zelle präsentiert werden.

21. Zusammensetzung, umfassend die Gastzelle gemäß Anspruch 19 oder 20 und einen Träger, vorzugsweise einen pharmazeutisch akzeptablen Träger.

22. Verwendung einer wirksamen Menge von zwei oder mehreren immunogenen Liganden, wobei jeder der immunogenen Liganden durch eine Fähigkeit, eine Immunantwort gegen den gleichen nativen Liganden auszulösen, charakterisiert ist, und wobei jeder der immunogenen Liganden aus der Gruppe ausgewählt ist, bestehend aus
FLTDERMPV (SEQ ID NO:3), FLTDEARSV (SEQ ID NO:5),
FLDDEIMRV (SEQ ID NO:7), FLDDEITFV (SEQ ID NO:9) und
IIEDDRLPV (SEQ ID NO:11)
zur Herstellung eines Medikaments zur Induktion einer Immunantwort in einem Subjekt.

## Revendications

1. Composé ayant la structure choisie dans le groupe de

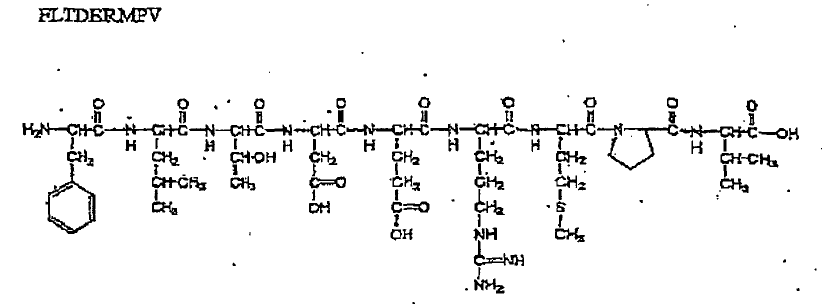

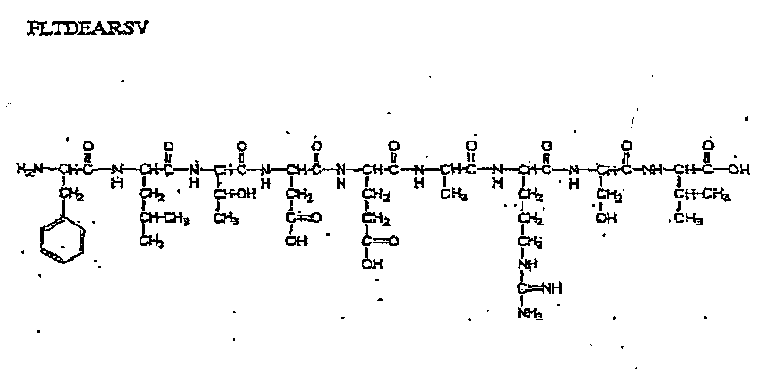

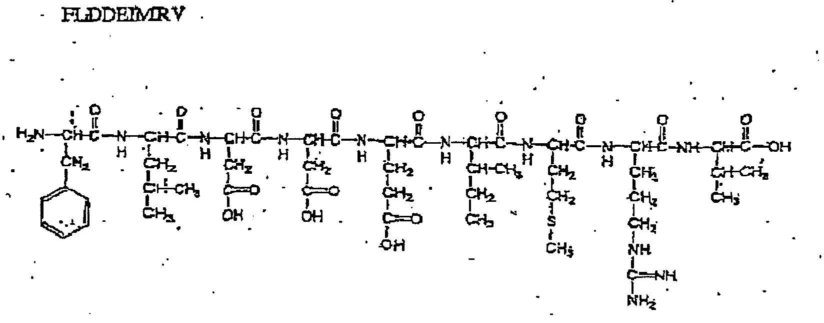

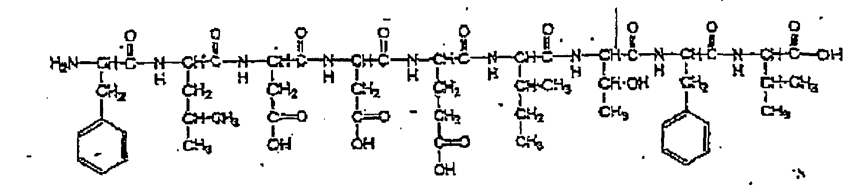

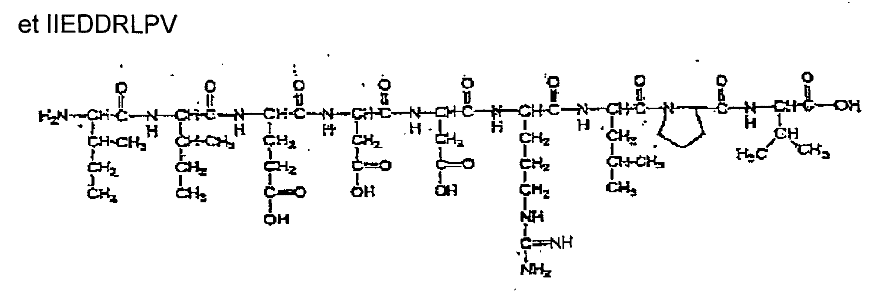

2. Peptide comprenant la séquence d'acides aminés de n° d'ID. SEQ. 2 où les acides aminés 700, 701, 702, 704 et 706 sont F, L, T, E, et M, respectivement.

3. Peptide comprenant la séquence d'acides aminés de n° d'ID. SEQ. 2, où les acides aminés 700, 701, 702, 704, 705, 706, et 707 sont F, L, D, E, I, T et F, respectivement, ou F, L, T, E, A, R, et S, respectivement ou F, L, D, E, I, M et R, respectivement.

4. Polynucléotide qui code une séquence d'acides aminés comprenant les acides aminés de n° d'ID. SEQ. 3, n° d'ID. SEQ. 5, n° d'ID. SEQ. 7 ou n° d'ID. SEQ. 9.

5. Anticorps qui reconnaît spécifiquement et se fixe à un composé selon la revendication 1.

6. Utilisation d'une quantité efficace d'un composé selon la revendication 1 pour la préparation d'un médicament pour déclencher une réponse immunitaire chez un sujet.

7. Utilisation selon la revendication 6, **caractérisée en ce que** le composé est délivré dans le contexte d'une molécule CMH, ou sous forme d'un polynucléotide qui code le composé.

8. Utilisation selon la revendication 6 ou 7, **caractérisée en ce que** la molécule CMH présente le composé sur la surface d'une cellule présentatrice d'antigène.

9. Utilisation d'une quantité efficace d'un anticorps selon la revendication 5 pour la préparation d'un médicament pour l'immunothérapie chez un sujet.

10. Cellule effectrice immunitaire qui a été élevée in vitro ou in vivo en présence de et aux dépens d'une cellule présentatrice d'antigène qui présente un composé selon la revendication 1 dans le contexte d'une molécule CMH.

11. Utilisation d'une quantité efficace de la cellule effectrice immunitaire selon la revendication 10 pour la préparation d'un médicament pour l'immunothérapie adoptive chez un sujet.

12. Composition comprenant au moins deux ligands immunogènes, **caractérisée en ce que** lesdits ligands immunogènes sont caractérisés individuellement par une aptitude à déclencher une réponse immunitaire contre le même ligand natif, et **caractérisée en ce que** ledit ligand immunogène est choisi dans le groupe comprenant FLTDERMPV (n° ID. SEQ. 3), FLTDEARSV (n° ID. SEQ. 5), FLDDEIMRV (n° ID. SEQ. 7), FLDDEITFV (n° ID. SEQ. 9) et IIEDDRLPV (n° ID. SEQ. 11).

13. Composition selon la revendication 12, comprenant de plus un domaine variable d'immunoglobuline biologiquement active lié auxdits ligands immunogènes.

14. Composition selon la revendication 12, comprenant de plus une molécule CMH liée auxdits ligands immunogènes.

15. Composition selon la revendication 12, **caractérisée en ce que** lesdits ligands immunogènes sont liés de façon covalente.

16. Composition selon la revendication 12, comprenant de plus un agent lié de façon covalente auxdits ligands immunogènes, **caractérisée en ce que** ledit agent est capable de cibler lesdits ligands immunogènes vers une cellule présentatrice d'antigène, de préférence une cellule dendritique.

17. Composition selon la revendication 12, comprenant de plus un peptide auxiliaire de fixation CMH de classe II.

18. Composition selon l'une des revendications 12 à 17, comprenant de plus un porteur, de préférence un porteur pharmaceutiquement acceptable.

19. Cellule hôte comprenant au moins deux ligands immunogènes, **caractérisée en ce que** lesdits ligands immunogènes sont individuellement **caractérisés par** une aptitude à déclencher une réponse immunitaire contre le même ligand natif, et **caractérisée en ce que** ledit ligand immunogène est choisi dans le groupe comprenant FLTDERMPV (n° ID. SEQ. 3), FLTDEARSV (n° ID. SEQ. 5), FLDDEIMRV (n° ID. SEQ. 7), FLDDEITFV (n° ID. SEQ. 9) et IIEDDRLPV (n° ID. SEQ. 11).

20. Cellule hôte selon la revendication 19, **caractérisée en ce que** la cellule hôte est une cellule présentatrice d'antigène, de préférence une cellule dendritique, et les ligands immunogènes sont présentés sur la surface de la cellule.

21. Composition comprenant la cellule hôte selon la revendication 19 ou 20, et un porteur, de préférence un porteur pharmaceutiquement acceptable.

22. Utilisation d'une quantité efficace de deux ou plusieurs ligands immunogènes, **caractérisée en ce que** chacun desdits ligands immunogènes est **caractérisé par** une aptitude à déclencher une réponse immunitaire contre le même ligand natif, et en ce que ledit ligand immunogène est choisi dans le groupe comprenant FLTDERMPV (n° ID. SEQ. 3), FLTDEARSV (n° ID. SEQ. 5), FLDDEIMRV (n° ID. SEQ. 7), FLDDEITFV (n° ID. SEQ. 9) et IIEDDRLPV (n° ID. SEQ. 11) pour la préparation d'un médicament pour déclencher une réponse immunitaire chez un sujet.


### SEQUENCE LISTING

<110> Genzyme Corporation
   Nicolette, Charles, A.
<120> THERAPEUTIC COMPOUNDS FOR OVARIAN CANCER
<130> 194422104.40
<140> Not Yet Assigned
   <141> - -
<150> US 60/226,243
   <151> 2000-08-17
<160> 12
<170> FastSEQ for Windows Version 4.0
<210> 1
   <211> 3801
   <212> DNA
   <213> Homo sapiens
<220>
   <221> CDS
   <222> (1)...(3801)
<400> 1
   atg aaa gtg acc gtg tgc ttc gga cgg acc cgg gtg gtc gtg ccg tgc      48
   Met Lys Val Thr Val Cys Phe Gly Arg Thr Arg Val Val Val Pro Cys
    1               5                  10                  15
   ggg gac ggc cac atg aaa gtt ttc agc ctc atc cag cag gcg gtg acc      96
   Gly Asp Gly His Met Lys Val Phe Ser Leu Ile Gln Gln Ala Val Thr
               20                  25                  30
   cgc tac cgg aag gcc atc gcc aag gat cca aac tac tgg ata cag gtg     144
   Arg Tyr Arg Lys Ala Ile Ala Lys Asp Pro Asn Tyr Trp Ile Gln Val
           35                  40                  45
   cat cgc ttg gaa cat gga gat gga gga ata cta gac ctt gat gac att     192
   His Arg Leu Glu His Gly Asp Gly Gly Ile Leu Asp Leu Asp Asp Ile
       50                  55                  60
   ctt tgt gat gta gca gac gat aaa gac aga ctg gta gca gtg ttt gat     240
   Leu Cys Asp Val Ala Asp Asp Lys Asp Arg Leu Val Ala Val Phe Asp
   65                  70                  75                  80
   gag cag gat cca cat cac gga ggt gat ggc acc agt gcc agt tcc acg     288
   Glu Gln Asp Pro His His Gly Gly Asp Gly Thr Ser Ala Ser Ser Thr
                   85                  90                  95
   ggt acc cag agc cca gag ata ttt ggt agt gag ctt ggc acc aac aat     336
   Gly Thr Gln Ser Pro Glu Ile Phe Gly Ser Glu Leu Gly Thr Asn Asn
               100                 105                 110
   gtc tca gcc ttt cag cct tac caa gca aca agt gaa att gag gtc aca     384
   Val Ser Ala Phe Gln Pro Tyr Gln Ala Thr Ser Glu Ile Glu Val Thr
           115                 120                 125
   cct tca gtc ctt cga gca aat atg cct ctt cat gtt cga cgc agt agt     432
   Pro Ser Val Leu Arg Ala Asn Met Pro Leu His Val Arg Arg Ser Ser
       130                 135                 140
   gac cca gct cta att ggc ctc tcc act tct gtc agt gat agt aat ttt     480
   Asp Pro Ala Leu Ile Gly Leu Ser Thr Ser Val Ser Asp Ser Asn Phe
   145                 150                 155                 160
   tcc tct gaa gag cct tca agg aaa aat ccc aca cgc tgg tca aca aca     528
   Ser Ser Glu Glu Pro Ser Arg Lys Asn Pro Thr Arg Trp Ser Thr Thr
                   165                 170                 175
   gct ggc ttc ctc aag cag aac act gct ggg agt cct aaa acc tgc gac     576
   Ala Gly Phe Leu Lys Gln Asn Thr Ala Gly Ser Pro Lys Thr Cys Asp
               180                 185                 190
   agg aag gat gag gat ggg aca gaa gag gat aac agt cgt gtt gaa cct     624
   Arg Lys Asp Glu Asp Gly Thr Glu Glu Asp Asn Ser Arg Val Glu Pro
           195                 200                 205
   gtt gga cat gct gac acg ggt ttg gag cat ata ccc aac ttt tct ctg     672
   Val Gly His Ala Asp Thr Gly Leu Glu His Ile Pro Asn Phe Ser Leu
       210                 215                 220
   gat gat atg gta aag ctc gta gaa gtc ccc aac gat gga ggg cct ctg     720
   Asp Asp Met Val Lys Leu Val Glu Val Pro Asn Asp Gly Gly Pro Leu
   225                 230                 235                 240
   gga atc cat gta gtg cct ttc agt gct cga ggc ggc aga acc ctg ggg     768
   Gly Ile His Val Val Pro Phe Ser Ala Arg Gly Gly Arg Thr Leu Gly
                   245                 250                 255
   tta tta gta aaa cga ttg gag aaa ggt ggt aaa gct gaa cat gaa aat     816
   Leu Leu Val Lys Arg Leu Glu Lys Gly Gly Lys Ala Glu His Glu Asn
               260                 265                 270
   ctt ttt cgt gag aat gat tgc att gtc agg att aat gat ggc gac ctt     864
   Leu Phe Arg Glu Asn Asp Cys Ile Val Arg Ile Asn Asp Gly Asp Leu
           275                 280                 285
   cga aat aga aga ttt gaa caa gca caa cat atg ttt cgc caa gcc atg     912
   Arg Asn Arg Arg Phe Glu Gln Ala Gln His Met Phe Arg Gln Ala Met
       290                 295                 300
   cgt aca ccc atc att tgg ttc cat gtg gtt cct gca gca aat aaa gag     960
   Arg Thr Pro Ile Ile Trp Phe His Val Val Pro Ala Ala Asn Lys Glu
   305                 310                 315                 320
   cag tat gaa caa cta tcc caa agt gag aag aac aat tac tat tca agc     1008
   Gln Tyr Glu Gln Leu Ser Gln Ser Glu Lys Asn Asn Tyr Tyr Ser Ser
                   325                 330                 335
   cgt ttt agc cct gac agc cag tat att gac aac agg agt gtg aac agt     1056
   Arg Phe Ser Pro Asp Ser Gln Tyr Ile Asp Asn Arg Ser Val Asn Ser
               340                 345                 350
   gca ggg ctt cac acg gtg cag aga gca ccc cga ctg aac cac ccg cct     1104
   Ala Gly Leu His Thr Val Gln Arg Ala Pro Arg Leu Asn His Pro Pro
           355                 360                 365
   gag cag ata gac tct cac tca aga cta cct cat agc gca cac ccc tcg     1152
   Glu Gln Ile Asp Ser His Ser Arg Leu Pro His Ser Ala His Pro Ser
       370                 375                 380
   gga aaa cca cca tcc gct cca gcc tcg gca cct cag aat gta ttt agt     1200
   Gly Lys Pro Pro Ser Ala Pro Ala Ser Ala Pro Gln Asn Val Phe Ser
   385                 390                 395                 400
   acg act gta agc agt ggt tat aac acc aaa aaa ata ggc aag agg ctt     1248
   Thr Thr Val Ser Ser Gly Tyr Asn Thr Lys Lys Ile Gly Lys Arg Leu
                   405                 410                 415
   aat atc cag ctt aag aaa ggt aca gaa ggt ttg gga ttc agc atc act     1296
   Asn Ile Gln Leu Lys Lys Gly Thr Glu Gly Leu Gly Phe Ser Ile Thr
               420                 425                 430
   tcc aga gat gta aca ata ggt ggc tca gct cca atc tat gtg aaa aac    1344
   Ser Arg Asp Val Thr Ile Gly Gly Ser Ala Pro Ile Tyr Val Lys Asn
           435                 440                 445
   att ctc ccc cgg ggg gcg gcc att cag gat ggc cga ctt aag gca gga    1392
   Ile Leu Pro Arg Gly Ala Ala Ile Gln Asp Gly Arg Leu Lys Ala Gly
       450                 455                 460
   gac aga ctt ata gag gta aat gga gta gat tta gtg ggc aaa tcc caa    1440
   Asp Arg Leu Ile Glu Val Asn Gly Val Asp Leu Val Gly Lys Ser Gln
   465                 470                 475                 480
   gag gaa gtt gtt tcg ctg ttg aga agc acc aag atg gaa gga act gtg    1488
   Glu Glu Val Val Ser Leu Leu Arg Ser Thr Lys Met Glu Gly Thr Val
                   485                 490                 495
   agc ctt ctg gtc ttt cgc cag gaa gac gcc ttc cac cca agg gaa ctg    1536
   Ser Leu Leu Val Phe Arg Gln Glu Asp Ala Phe His Pro Arg Glu Leu
               500                 505                 510
   aaa gca gaa gat gag gat att gtt ctt aca cct gat ggc acc agg gaa    1584
   Lys Ala Glu Asp Glu Asp Ile Val Leu Thr Pro Asp Gly Thr Arg Glu
           515                 520                 525
   ttt ctg aca ttt gaa gtc cca ctt aat gat tca gga tct gca ggc ctt    1632
   Phe Leu Thr Phe Glu Val Pro Leu Asn Asp Ser Gly Ser Ala Gly Leu
       530                 535                 540
   ggt gtc agt gtc aaa ggt aac cgg tca aaa gag aac cac gca gat ttg    1680
   Gly Val Ser Val Lys Gly Asn Arg Ser Lys Glu Asn His Ala Asp Leu
   545                 550                 555                 560
   gga atc ttt gtc aag tcc att att aat gga gga gca gca tct aaa gat    1728
   Gly Ile Phe Val Lys Ser Ile Ile Asn Gly Gly Ala Ala Ser Lys Asp
                   565                 570                 575
   gga agg ctt cgg gtg aat gat caa ctg ata gca gta aat gga gaa tcc    1776
   Gly Arg Leu Arg Val Asn Asp Gln Leu Ile Ala Val Asn Gly Glu Ser
               580                 585                 590
   ctg ttg ggc aag aca aac caa gat gcc atg gaa acc cta aga agg tct    1824
   Leu Leu Gly Lys Thr Asn Gln Asp Ala Met Glu Thr Leu Arg Arg Ser
           595                 600                 605
   atg tct act gaa ggc aat aaa cga gga atg atc cag ctt att gtt gca    1872
   Met Ser Thr Glu Gly Asn Lys Arg Gly Met Ile Gln Leu Ile Val Ala
       610                 615                 620
   agg aga ata agc aag tgc aat gag ctg aag tca cct ggg agc ccc cct    1920
   Arg Arg Ile Ser Lys Cys Asn Glu Leu Lys Ser Pro Gly Ser Pro Pro
   625                 630                 635                 640
   gga cct gag ctg ccc att gaa aca gcg ttg gat gat aga gaa cga aga    1968
   Gly Pro Glu Leu Pro Ile Glu Thr Ala Leu Asp Asp Arg Glu Arg Arg
                   645                 650                 655
   att tcc cat tcc ctc tac agt ggg att gag ggg ctt gat gaa tcc cca    2016
   Ile Ser His Ser Leu Tyr Ser Gly Ile Glu Gly Leu Asp Glu Ser Pro
               660                 665                 670
   agc aga aat gct gcc ctc agt agg ata atg ggt aaa tac cag ctg tcc    2064
   Ser Arg Asn Ala Ala Leu Ser Arg Ile Met Gly Lys Tyr Gln Leu Ser
           675                 680                 685
   cct aca gtg aat atg ccc caa gat gac act gtc att ata gaa gat gac    2112
   Pro Thr Val Asn Met Pro Gln Asp Asp Thr Val Ile Ile Glu Asp Asp
       690                 695                 700
   agg ttg cca gtg ctt cct cca cat ctc tct gac cag tcc tct tcc agc      2160
   Arg Leu Pro Val Leu Pro Pro His Leu Ser Asp Gln Ser Ser Ser Ser
   705                 710                 715                 720
   tcc cat gat gat gtg ggg ttt gtg acg gca gat gct ggt act tgg gcc      2208
   Ser His Asp Asp Val Gly Phe Val Thr Ala Asp Ala Gly Thr Trp Ala
                   725                 730                 735
   aag gct gca atc agt gat tca gcc gac tgc tct ttg agt cca gat gtt      2256
   Lys Ala Ala Ile Ser Asp Ser Ala Asp Cys Ser Leu Ser Pro Asp Val
               740                 745                 750
   gat cca gtt ctt gct ttt caa cga gaa gga ttt gga cgt cag ata gct      2304
   Asp Pro Val Leu Ala Phe Gln Arg Glu Gly Phe Gly Arg Gln Ile Ala
           755                 760                 765
   gac gag act aaa ctc aat aca gtg gat gac cag aaa gca ggt tct ccc      2352
   Asp Glu Thr Lys Leu Asn Thr Val Asp Asp Gln Lys Ala Gly Ser Pro
       770                 775                 780
   agc aga gat gtg ggt cct tcc ctg ggt ctg aag aag tca agc tcg ttg      2400
   Ser Arg Asp Val Gly Pro Ser Leu Gly Leu Lys Lys Ser Ser Ser Leu
   785                 790                 795                 800
   gag agt ctg cag acc gca gtt gcc gag gtg act ttg aat ggg gat att      2448
   Glu Ser Leu Gln Thr Ala Val Ala Glu Val Thr Leu Asn Gly Asp Ile
                   805                 810                 815
   cct ttc cat cgt cca cgg ccg cgg ata atc aga ggc agg gga tgc aat      2496
   Pro Phe His Arg Pro Arg Pro Arg Ile Ile Arg Gly Arg Gly Cys Asn
               820                 825                 830
   gag agc ttc aga gct gcc atc gac aaa tct tat gat aaa ccc gcg gta      2544
   Glu Ser Phe Arg Ala Ala Ile Asp Lys Ser Tyr Asp Lys Pro Ala Val
           835                 840                 845
   gat gat gat gat gag ggc atg gag acc ttg gaa gaa gac aca gaa gaa      2592
   Asp Asp Asp Asp Glu Gly Met Glu Thr Leu Glu Glu Asp Thr Glu Glu
       850                 855                 860
   agt tca aga tca ggg aga gag tct gta tcc aca gcc agt gat cag cct      2640
   Ser Ser Arg Ser Gly Arg Glu Ser Val Ser Thr Ala Ser Asp Gln Pro
   865                 870                 875                 880
   tcc cac tct ctg gag aga caa atg aat gga aac caa gag aaa ggt gat      2688
   Ser His Ser Leu Glu Arg Gln Met Asn Gly Asn Gln Glu Lys Gly Asp
                   885                 890                 895
   aag act gat aga aaa aag gat aaa act gga aaa gaa aag aag aaa gat      2736
   Lys Thr Asp Arg Lys Lys Asp Lys Thr Gly Lys Glu Lys Lys Lys Asp
               900                 905                 910
   aga gat aag gag aag gat aaa atg aaa gcc aag aag gga atg ctg aag      2784
   Arg Asp Lys Glu Lys Asp Lys Met Lys Ala Lys Lys Gly Met Leu Lys
           915                 920                 925
   ggc ttg gga gac atg ttc agg ttt ggc aaa cat cga aaa gat gac aag      2832
   Gly Leu Gly Asp Met Phe Arg Phe Gly Lys His Arg Lys Asp Asp Lys
       930                 935                 940
   att gag aaa acg ggt aaa ata aaa ata cag gaa tcc ttt aca tca gaa      2880
   Ile Glu Lys Thr Gly Lys Ile Lys Ile Gln Glu Ser Phe Thr Ser Glu
   945                 950                 955                 960
   gag gag agg ata cga atg aag cag gag cag gag agg att caa gcc aaa      2928
   Glu Glu Arg Ile Arg Met Lys Gln Glu Gln Glu Arg Ile Gln Ala Lys
                   965                 970                 975
   act cga gaa ttt agg gaa cga caa gct cga gag cgt gac tat gct gaa     2976
   Thr Arg Glu Phe Arg Glu Arg Gln Ala Arg Glu Arg Asp Tyr Ala Glu
               980                 985                 990
   att caa gat ttt cat cgg aca ttt ggc tgt gat gat gag tta atg tat     3024
   Ile Gln Asp Phe His Arg Thr Phe Gly Cys Asp Asp Glu Leu Met Tyr
           995                 1000                1005
   ggg gga gtt tct tct tat gaa ggt tcc atg gct ctc aac gct aga cct     3072
   Gly Gly Val Ser Ser Tyr Glu Gly Ser Met Ala Leu Asn Ala Arg Pro
       1010                1015                1020
   cag agc cca cga gaa ggg cat atg atg gat gct ttg tat gcc caa gtc     3120
   Gln Ser Pro Arg Glu Gly His Met Met Asp Ala Leu Tyr Ala Gln Val
   1025                1030                1035                1040
   aag aag ccg cgg aat tcc aaa ccc tca cct gta gac agt aac aga tca     3168
   Lys Lys Pro Arg Asn Ser Lys Pro Ser Pro Val Asp Ser Asn Arg Ser
                   1045                1050                1055
   act cct agc aat cat gat cgg ata cag cgt ctg agg caa gaa ttt cag     3216
   Thr Pro Ser Asn His Asp Arg Ile Gln Arg Leu Arg Gln Glu Phe Gln
               1060                1065                1070
   caa gca aag caa gat gaa gat gta gaa gat cgt cgg cgg acc tat agt     3264
   Gln Ala Lys Gln Asp Glu Asp Val Glu Asp Arg Arg Arg Thr Tyr Ser
           1075                1080                1085
   ttt gag caa ccc tgg ccg aac gca cgg ccg gcg acg cag agc ggg cga     3312
   Phe Glu Gln Pro Trp Pro Asn Ala Arg Pro Ala Thr Gln Ser Gly Arg
       1090                1095                1100
   cac tcg gtg tcc gtg gag gtg cag atg cag cgg cag cgg cag gag gag     3360
   His Ser Val Ser Val Glu Val Gln Met Gln Arg Gln Arg Gln Glu Glu
   1105                1110                1115                1120
   cgc gag agc tcc cag cag gcc cag cgc cag tac agc tct ctg cct cgg     3408
   Arg Glu Ser Ser Gln Gln Ala Gln Arg Gln Tyr Ser Ser Leu Pro Arg
                   1125                1130                1135
   caa agc agg aaa aat gcc agc tcg gtc tcc cag gac tct tgg gag cag     3456
   Gln Ser Arg Lys Asn Ala Ser Ser Val Ser Gln Asp Ser Trp Glu Gln
               1140                1145                1150
   aac tac tcc cct ggg gaa ggc ttc cag agt gcc aaa gag aac ccc agg     3504
   Asn Tyr Ser Pro Gly Glu Gly Phe Gln Ser Ala Lys Glu Asn Pro Arg
           1155                1160                1165
   tac tcc agc tac caa ggc tcc agg aac ggc tac ctg gga gga cat ggc     3552
   Tyr Ser Ser Tyr Gln Gly Ser Arg Asn Gly Tyr Leu Gly Gly His Gly
       1170                1175                1180
   ttc aac gcc agg gtc atg ctg gaa act cag gag ctc ctt cgc cag gaa     3600
   Phe Asn Ala Arg Val Met Leu Glu Thr Gln Glu Leu Leu Arg Gln Glu
   1185                1190                1195                1200
   cag agg cgg aag gag cag cag atg aag aag cag cct cct tcc gag ggg     3648
   Gln Arg Arg Lys Glu Gln Gln Met Lys Lys Gln Pro Pro Ser Glu Gly
                   1205                1210                1215
   ccc agc aac tat gac tcg tat aag aaa gtc cag gac ccc agt tac gcc     3696
   Pro Ser Asn Tyr Asp Ser Tyr Lys Lys Val Gln Asp Pro Ser Tyr Ala
               1220                1225                1230
   cct ccc aag ggg ccc ttc cgg caa gat gtg ccc ccc tcc cct tct cag     3744
   Pro Pro Lys Gly Pro Phe Arg Gln Asp Val Pro Pro Ser Pro Ser Gln
           1235                1240                1245
   gtt gcg agg ctg aac aga ctt cag act cct gag aaa ggg agg ccc ttc      3792
   Val Ala Arg Leu Asn Arg Leu Gln Thr Pro Glu Lys Gly Arg Pro Phe
       1250                1255                1260
   tat tcc tga                                                          3801
   Tyr Ser  *
   1265
<210> 2
   <211> 1266
   <212> PRT
   <213> Homo sapiens
<400> 2
   Met Lys Val Thr Val Cys Phe Gly Arg Thr Arg Val Val Val Pro Cys
   1 5 10 15
   Gly Asp Gly His Met Lys Val Phe Ser Leu Ile Gln Gln Ala Val Thr
   20 25 30
   Arg Tyr Arg Lys Ala Ile Ala Lys Asp Pro Asn Tyr Trp Ile Gln Val
   35 40 45
   His Arg Leu Glu His Gly Asp Gly Gly Ile Leu Asp Leu Asp Asp Ile
   50 55 60
   Leu Cys Asp Val Ala Asp Asp Lys Asp Arg Leu Val Ala Val Phe Asp
   65 70 75 80
   Glu Gln Asp Pro His His Gly Gly Asp Gly Thr Ser Ala Ser Ser Thr
   85 90 95
   Gly Thr Gln Ser Pro Glu Ile Phe Gly Ser Glu Leu Gly Thr Asn Asn
   100 105 110
   Val Ser Ala Phe Gln Pro Tyr Gln Ala Thr Ser Glu Ile Glu Val Thr
   115 120 125
   Pro Ser Val Leu Arg Ala Asn Met Pro Leu His Val Arg Arg Ser Ser
   130 135 140
   Asp Pro Ala Leu Ile Gly Leu Ser Thr Ser Val Ser Asp Ser Asn Phe
   145 150 155 160
   Ser Ser Glu Glu Pro Ser Arg Lys Asn Pro Thr Arg Trp Ser Thr Thr
   165 170 175
   Ala Gly Phe Leu Lys Gln Asn Thr Ala Gly Ser Pro Lys Thr Cys Asp
   180 185 190
   Arg Lys Asp Glu Asp Gly Thr Glu Glu Asp Asn Ser Arg Val Glu Pro
   195 200 205
   Val Gly His Ala Asp Thr Gly Leu Glu His Ile Pro Asn Phe Ser Leu
   210 215 220
   Asp Asp Met Val Lys Leu Val Glu Val Pro Asn Asp Gly Gly Pro Leu
   225 230 235 240
   Gly Ile His Val Val Pro Phe Ser Ala Arg Gly Gly Arg Thr Leu Gly
   245 250 255
   Leu Leu Val Lys Arg Leu Glu Lys Gly Gly Lys Ala Glu His Glu Asn
   260 265 270
   Leu Phe Arg Glu Asn Asp Cys Ile Val Arg Ile Asn Asp Gly Asp Leu
   275 280 285
   Arg Asn Arg Arg Phe Glu Gln Ala Gln His Met Phe Arg Gln Ala Met
   290 295 300
   Arg Thr Pro Ile Ile Trp Phe His Val Val Pro Ala Ala Asn Lys Glu
   305 310 315 320
   Gln Tyr Glu Gln Leu Ser Gln Ser Glu Lys Asn Asn Tyr Tyr Ser Ser
   325 330 335
   Arg Phe Ser Pro Asp Ser Gln Tyr Ile Asp Asn Arg Ser Val Asn Ser
   340 345 350
   Ala Gly Leu His Thr Val Gln Arg Ala Pro Arg Leu Asn His Pro Pro
   355 360 365
   Glu Gln Ile Asp Ser His Ser Arg Leu Pro His Ser Ala His Pro Ser
   370 375 380
   Gly Lys Pro Pro Ser Ala Pro Ala Ser Ala Pro Gln Asn Val Phe Ser
   385 390 395 400
   Thr Thr Val Ser Ser Gly Tyr Asn Thr Lys Lys Ile Gly Lys Arg Leu
                   405                 410                 415
   Asn Ile Gln Leu Lys Lys Gly Thr Glu Gly Leu Gly Phe Ser Ile Thr
               420                 425                 430
   Ser Arg Asp Val Thr Ile Gly Gly Ser Ala Pro Ile Tyr Val Lys Asn
           435                 440                 445
   Ile Leu Pro Arg Gly Ala Ala Ile Gln Asp Gly Arg Leu Lys Ala Gly
       450                 455                 460
   Asp Arg Leu Ile Glu Val Asn Gly Val Asp Leu Val Gly Lys Ser Gln
   465                 470                 475                 480
   Glu Glu Val Val Ser Leu Leu Arg Ser Thr Lys Met Glu Gly Thr Val
                   485                 490                 495
   Ser Leu Leu Val Phe Arg Gln Glu Asp Ala Phe His Pro Arg Glu Leu
               500                 505                 510
   Lys Ala Glu Asp Glu Asp Ile Val Leu Thr Pro Asp Gly Thr Arg Glu
           515                 520                 525
   Phe Leu Thr Phe Glu Val Pro Leu Asn Asp Ser Gly Ser Ala Gly Leu
       530                 535                 540
   Gly Val Ser Val Lys Gly Asn Arg Ser Lys Glu Asn His Ala Asp Leu
   545                 550                 555                 560
   Gly Ile Phe Val Lys Ser Ile Ile Asn Gly Gly Ala Ala Ser Lys Asp
                   565                 570                 575
   Gly Arg Leu Arg Val Asn Asp Gln Leu Ile Ala Val Asn Gly Glu Ser
               580                 585                 590
   Leu Leu Gly Lys Thr Asn Gln Asp Ala Met Glu Thr Leu Arg Arg Ser
           595                 600                 605
   Met Ser Thr Glu Gly Asn Lys Arg Gly Met Ile Gln Leu Ile Val Ala
       610                 615                 620
   Arg Arg Ile Ser Lys Cys Asn Glu Leu Lys Ser Pro Gly Ser Pro Pro
   625                 630                 635                 640
   Gly Pro Glu Leu Pro Ile Glu Thr Ala Leu Asp Asp Arg Glu Arg Arg
                   645                 650                 655
   Ile Ser His Ser Leu Tyr Ser Gly Ile Glu Gly Leu Asp Glu Ser Pro
               660                 665                 670
   Ser Arg Asn Ala Ala Leu Ser Arg Ile Met Gly Lys Tyr Gln Leu Ser
           675                 680                 685
   Pro Thr Val Asn Met Pro Gln Asp Asp Thr Val Ile Ile Glu Asp Asp
       690                 695                 700
   Arg Leu Pro Val Leu Pro Pro His Leu Ser Asp Gln Ser Ser Ser Ser
   705                 710                 715                 720
   Ser His Asp Asp Val Gly Phe Val Thr Ala Asp Ala Gly Thr Trp Ala
                   725                 730                 735
   Lys Ala Ala Ile Ser Asp Ser Ala Asp Cys Ser Leu Ser Pro Asp Val
               740                 745                 750
   Asp Pro Val Leu Ala Phe Gln Arg Glu Gly Phe Gly Arg Gln Ile Ala
           755                 760                 765
   Asp Glu Thr Lys Leu Asn Thr Val Asp Asp Gln Lys Ala Gly Ser Pro
       770                 775                 780
   Ser Arg Asp Val Gly Pro Ser Leu Gly Leu Lys Lys Ser Ser Ser Leu
   785                 790                 795                 800
   Glu Ser Leu Gln Thr Ala Val Ala Glu Val Thr Leu Asn Gly Asp Ile
                   805                 810                 815
   Pro Phe His Arg Pro Arg Pro Arg Ile Ile Arg Gly Arg Gly Cys Asn
               820                 825                 830
   Glu Ser Phe Arg Ala Ala Ile Asp Lys Ser Tyr Asp Lys Pro Ala Val
           835                 840                 845
   Asp Asp Asp Asp Glu Gly Met Glu Thr Leu Glu Glu Asp Thr Glu Glu
       850                 855                 860
   Ser Ser Arg Ser Gly Arg Glu Ser Val Ser Thr Ala Ser Asp Gln Pro
   865                 870                 875                 880
   Ser His Ser Leu Glu Arg Gln Met Asn Gly Asn Gln Glu Lys Gly Asp
                   885                 890                 895
   Lys Thr Asp Arg Lys Lys Asp Lys Thr Gly Lys Glu Lys Lys Lys Asp
               900                 905                 910
   Arg Asp Lys Glu Lys Asp Lys Met Lys Ala Lys Lys Gly Met Leu Lys
           915                 920                 925
   Gly Leu Gly Asp Met Phe Arg Phe Gly Lys His Arg Lys Asp Asp Lys
       930                 935                 940
   Ile Glu Lys Thr Gly Lys Ile Lys Ile Gln Glu Ser Phe Thr Ser Glu
   945 950 955 960
   Glu Glu Arg Ile Arg Met Lys Gln Glu Gln Glu Arg Ile Gln Ala Lys
   965 970 975
   Thr Arg Glu Phe Arg Glu Arg Gln Ala Arg Glu Arg Asp Tyr Ala Glu
   980 985 990
   Ile Gln Asp Phe His Arg Thr Phe Gly Cys Asp Asp Glu Leu Met Tyr
   995 1000 1005
   Gly Gly Val Ser Ser Tyr Glu Gly Ser Met Ala Leu Asn Ala Arg Pro
   1010 1015 1020
   Gln Ser Pro Arg Glu Gly His Met Met Asp Ala Leu Tyr Ala Gln Val
   1025 1030 1035 1040
   Lys Lys Pro Arg Asn Ser Lys Pro Ser Pro Val Asp Ser Asn Arg Ser
   1045 1050 1055
   Thr Pro Ser Asn His Asp Arg Ile Gln Arg Leu Arg Gln Glu Phe Gln
   1060 1065 1070
   Gln Ala Lys Gln Asp Glu Asp Val Glu Asp Arg Arg Arg Thr Tyr Ser
   1075 1080 1085
   Phe Glu Gln Pro Trp Pro Asn Ala Arg Pro Ala Thr Gln Ser Gly Arg
   1090 1095 1100
   His Ser Val Ser Val Glu Val Gln Met Gln Arg Gln Arg Gln Glu Glu
   1105 1110 1115 1120
   Arg Glu Ser Ser Gln Gln Ala Gln Arg Gln Tyr Ser Ser Leu Pro Arg
   1125 1130 1135
   Gln Ser Arg Lys Asn Ala Ser Ser Val Ser Gln Asp Ser Trp Glu Gln
   1140 1145 1150
   Asn Tyr Ser Pro Gly Glu Gly Phe Gln Ser Ala Lys Glu Asn Pro Arg
   1155 1160 1165
   Tyr Ser Ser Tyr Gln Gly Ser Arg Asn Gly Tyr Leu Gly Gly His Gly
   1170 1175 1180
   Phe Asn Ala Arg Val Met Leu Glu Thr Gln Glu Leu Leu Arg Gln Glu
   1185 1190 1195 1200
   Gln Arg Arg Lys Glu Gln Gln Met Lys Lys Gln Pro Pro Ser Glu Gly
   1205 1210 1215
   Pro Ser Asn Tyr Asp Ser Tyr Lys Lys Val Gln Asp Pro Ser Tyr Ala
   1220 1225 1230
   Pro Pro Lys Gly Pro Phe Arg Gln Asp Val Pro Pro Ser Pro Ser Gln
   1235 1240 1245
   Val Ala Arg Leu Asn Arg Leu Gln Thr Pro Glu Lys Gly Arg Pro Phe
   1250 1255 1260
   Tyr Ser
   1265
<210> 3
   <211> 9
   <212> PRT
   <213> Homo sapiens
<400> 3
   Pro Leu Thr Asp Glu Arg Met Pro Val
   1 5
<210> 4
   <211> 27
   <212> DNA
   <213> Homo sapiens
<220>
   <221> misc_feature
   <222> 6, 9, 18, 24, 27
   <223> n = A,T,C or G
<400> 4
   ttyctnacng aygarcgnat gccngtn 27
<210> 5
   <211> 9
   <212> PRT
   <213> Homo sapiens
<400> 5
   Phe Leu Thr Asp Glu Ala Arg Ser Val
   1 5
<210> 6
   <211> 27
   <212> DNA
   <213> Homo sapiens
<220>
   <221> misc_feature
   <222> 6, 9, 18, 21, 24, 27
   <223> n = A,T,C or G
<400> 6
   ttyctnacng aygargcnmg nwsngtn 27
<210> 7
   <211> 9
   <212> PRT
   <213> Homo sapiens
<400> 7
   Phe Leu Asp Asp Glu Ile Met Arg Val
   1 5
<210> 8
   <211> 27
   <212> DNA
   <213> Homo sapiens
<220>
   <221> misc_feature
   <222> 6, 24, 27
   <223> n = A,T,C or G
<400> 8
   ttyctngayg aygarathat gmgngtn 27
<210> 9
   <211> 9
   <212> PRT
   <213> Homo sapiens
<400> 9
   Phe Leu Asp Asp Glu Ile Thr Phe Val
   1               5
<210> 10
   <211> 27
   <212> DNA
   <213> Homo sapiens
<220>
   <221> misc_feature
   <222> 6, 21, 27
   <223> n = A,T,C or G
<400> 10
   ttyctngayg aygarathac nttygtn 27
<210> 11
   <211> 9
   <212> PRT
   <213> Homo sapiens
<400> 11
   Ile Ile Glu Asp Asp Arg Leu Pro Val
   1               5
<210> 12
   <211> 27
   <212> DNA
   <213> Homo sapiens
<220>
   <221> misc_feature
   <222> 18, 21, 24, 27
   <223> n = A,T,C or G
<400> 12
   athathgarg aygaycgnyt nccngtn 27